(19) Europäisches Patentamt
European Patent Office
Office européen des brevets

(11) Veröffentlichungsnummer : **0 344 110 B1**

# (12) EUROPÄISCHE PATENTSCHRIFT

(45) Veröffentlichungstag der Patentschrift : **16.09.92 Patentblatt 92/38**

(51) Int. Cl.$^5$ : **C07C 323/22,** G03C 1/72, C07C 255/56, C07C 235/70, C07C 50/22

(21) Anmeldenummer : **89810364.3**

(22) Anmeldetag : **18.05.89**

## (54) Substituierte Naphthacen-5,12-dione und deren Verwendung.

(30) Priorität : **27.05.88 CH 2008/88**
**19.07.88 CH 2756/88**

(43) Veröffentlichungstag der Anmeldung : **29.11.89 Patentblatt 89/48**

(45) Bekanntmachung des Hinweises auf die Patenterteilung :
**16.09.92 Patentblatt 92/38**

(84) Benannte Vertragsstaaten :
**CH DE FR GB IT LI NL**

(56) Entgegenhaltungen :
**CHEMICAL ABSTRACTS, Band 83, Nr. 14, 6. Oktober 1975, Seite 65, Zusammenfassung Nr. 115804w, Columbus, Ohio, US; &JP-A-74 81 440**

(73) Patentinhaber : **CIBA-GEIGY AG**
**Klybeckstrasse 141**
**CH-4002 Basel (CH)**

(72) Erfinder : **Fischer, Walter, Dr.**
**Vogesenstrasse 77**
**CH-4153 Reinach (CH)**
Erfinder : **Baumann, Marcus, Dr.**
**Arlesheimerstrasse 18**
**CH-4053 Basel (CH)**
Erfinder : **Finter, Jürgen, Dr.**
**Zasiusstrasse 100**
**W-7800 Freiburg (DE)**
Erfinder : **Kvita, Vratislav, Dr.**
**Vogesenstrasse 71**
**CH-4153 Reinach (CH)**
Erfinder : **Mayer, Carl W., Dr.**
**Steingrubenweg 224**
**CH-4125 Riehen (CH)**
Erfinder : **Rembold, Manfred, Dr.**
**Im Aeschfeld 21**
**CH-4147 Aesch (CH)**
Erfinder : **Roth, Martin, Dr.**
**Oberdorf**
**CH-1735 Giffers (CH)**

EP 0 344 110 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft mit einem organischen Thiorest substituierte Naphthacen-5,12-dione; ein Verfahren zu deren Herstellung durch Diels-Alder-Reaktion von Benzocyclobuten-1,2-dihalogeniden mit entsprechend substituierten Naphtho-1,4-chinonen; eine durch Strahlung polymerisierbare Zusammensetzung, die ein solches Naphthacen-5,12-dion als Photoinitiator oder Sensibilisator enthalten; und die Verwendung dieser Zusammensetzung zur Herstellung von Lacken, Druckfarben, Druckplatten, Resistmaterialien sowie als Bildaufzeichungsmaterial und Beschichtungsmaterial.

In der JP-OS 49/081440 sind in 4-Stellung mit Phenylthio, p-Toluylthio oder p-Chlorphenylthio substituierte 1-Hydroxynaphthacen-5,12-dione als Fluoreszensfarbstoffe für Kunststoffe beschrieben. In der US-PS 3,941,759 ist Naphthacen-5,12-dion als Sensibilisatorkomponente für durch UV-Licht zersetzbare Kunststoffe beschrieben.

Es wurde nun gefunden, dass mit einem organischen Thiorest substituierte Naphthacen-5,12-dione ausgezeichnete Photoinitiatoren für die Photopolymerisation und gleichzeitig ausgezeichnete Sensibilisatoren für die Photodimerisation von ethylenisch ungesättigten Verbindungen sind.

Ein Gegenstand der Erfindung sind Verbindungen der Formel I

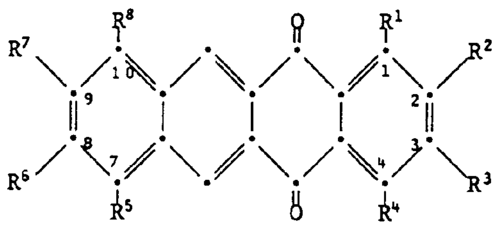

(I)

worin

$R^1$, $R^2$, $R^3$, $R^4$, $R^5$, $R^6$, $R^7$ und $R^8$ unabhängig voneinander H, -CN, -$NO_2$, Halogen, -$NR^{10}R^{11}$, -$COOR^9$, -$CONR^{10}R^{11}$, -$SiR^{13}R^{14}R^{15}$, oder unsubstituiertes oder mit -OH, -CN, -$NR^{10}R^{11}$, Halogen, $C_1$-$C_{18}$-Alkyl, -Alkoxy oder -Alkylthio, -$COOR^9$ oder -$CONR^{10}R^{11}$ substituiertes $C_1$-$C_{20}$-Alkyl$(X)_p$ $C_2$-$C_{20}$-Alkenyl$(X)_n$ $C_2$-$C_{18}$-Alkinyl$(X)_p$ $C_3$-$C_8$-Cycloalkyl$(X)_p$ $C_6$-$C_{12}$-Aryl$(X)_p$ oder $C_7$-$C_{14}$-Aralkyl$(X)_p$ darstellt oder -$Y(C_mH_{2m}O)_n$ $R^{12}$ ist;

X für O, S, SO oder $SO_2$ und p für 0 oder 1 stehen; Y für O oder S steht;

$R^9$ H oder den um ein Hydroxylwasserstoffatom verminderten Rest eines $C_1$-$C_{20}$-Alkohols und $R^{10}$ und $R^{11}$ unabhängig voneinander H, $C_1$-$C_{18}$-Alkyl, Phenyl, Benzyl, Cyclopentyl, Cyclohexyl, $C_2$-$C_{12}$-Mono- oder $C_2$-$C_{12}$-Dihydroxyalkyl, $(C_mH_{2m}O)_n$ $R^{12}$ oder $R^{10}$ und $R^{11}$ zusammen Tetra- oder Pentamethylen oder 3-Oxa-1,5-pentylen bedeuten; m 2 bis 6, n 2 bis 20 bedeuten; $R^{12}$ für H oder $C_1$-$C_{12}$-Alkyl steht; $R^{13}$, $R^{14}$ und $R^{15}$ unabhängig voneinander $C_1$-$C_{12}$-Alkyl darstellen; und wobei mindestens einer der Reste $R^2$, $R^3$, $R^5$, $R^6$, $R^7$ und $R^8$ einen Thiorest bedeuten.

Die Gruppen $R^1$ bis $R^8$ können ein- oder mehrfach, bevorzugt ein- bis sechsfach, besonders ein- bis dreifach und insbesondere ein- oder zweifach substituiert sein.

Wenn der Substituent Halogen bedeutet, so handelt es sich bevorzugt um -F, -Cl und -Br. Bedeutet der Substituent Alkyl, Alkoxy oder Alkylthio, so können diese Gruppen linear oder verzweigt sein und sie enthalten bevorzugt 1 bis 12, besonders 1 bis 6 C-Atome. Einige Beispiele sind Methyl, Ethyl, die Isomeren von Propyl, Butyl, Pentyl, Hexyl, Heptyl, Octyl, Nonyl, Decyl, Undecyl, Dodecyl, Tetradecyl, Octadecyl, sowie entsprechende Alkoxy- und Alkylthiogruppen.

Bei den Substituenten kann es sich um einen Rest der Formeln -$NR^{10}R^{11}$ oder -$CONR^{10}R^{11}$ handeln. $R^{10}$ und $R^{11}$ können hierin als Alkyl linear oder verzweigt sein und enthalten bevorzugt 1 bis 12, besonders 1 bis 8 C-Atome. In der Bedeutung von Monohydroxyalkyl enthalten $R^{10}$ und $R^{11}$ bevorzugt 2 bis 6 C-Atome; es kann sich um lineares oder verzweigtes Hydroxyalkyl handeln. Einige Beispiele sind 2-Hydroxyethyl, 2- oder 3-Hydroxyprop-1-yl, 2-, 3- oder 4-Hydroxybut-1-yl, 2-Hydroxybut-3-yl, Hydroxypentyl und Hydroxyhexyl. In der Bedeutung von Dihydroxyalkyl können $R^{10}$ und $R^{11}$ linear oder verzweigt sein und enthalten bevorzugt 3 bis 6 C-Atome. Beispiele sind 1,2-Dihydroxyprop-3-yl, 1,2-Dihydroxybut-3- oder -4-yl. $R^{10}$ und $R^{11}$ können einen Rest der Formel $(C_mH_{2m}O)_n$ $R^{12}$ darstellen, worin m vorzugsweise eine Zahl von 2 bis 4, besonders 2 oder 3, n bevorzugt eine Zahl von 2 bis 12, besonders 2 bis 6 und $R^{12}$ H oder lineares oder verzweigtes Alkyl mit vorzugsweise 1 bis 6 C-Atomen bedeuten.

Bei den Substituenten kann es sich um die Gruppe -$COOR^9$ handeln, worin $R^9$ H oder der um ein Hydroxylwasserstoffatom verminderte Rest eines Alkohols mit vorzugsweise 1 bis 12, besonders 1 bis 8 C-Atomen ist. $R^9$ kann z.B. lineares oder verzweigtes $C_1$-$C_{20}$-, bevorzugt $C_1$-$C_{12}$- und besonders $C_1$-$C_8$-Alkyl, Cyclopentyl, Cyclohexyl, unsubstituiertes oder mit $C_1$-$C_{12}$-Alkyl substituiertes Phenyl oder Benzyl oder der Rest der Formel $-(C_mH_{2m}O)_n-$ $R^{12}$ sein, wobei $R^{12}$, m und n die zuvor angegebenen Bedeutungen haben, einschliesslich der Bevorzugungen.

In einer bevorzugten Ausführungsform handelt es sich bei dem Substituenten für $R^1$ bis $R^8$ um -OH, -F, -Cl, -Br, $-NR^{10}R^{11}$, $C_1$-$C_{12}$-Alkyl, $C_1$-$C_{12}$-Alkoxy, $C_1$-$C_{12}$-Alkylthio oder $-COOR^9$, wobei $R^9$ für H oder $C_1$-$C_{18}$-Alkyl und $R^{10}$ und $R^{11}$ unabhängig voneinander für H, $C_1$-$C_{18}$-Alkyl, $-CH_2CH_2OH$, $-CH_2CH(OH)CH_3$, $-CH_2CHOHCH_2OH$, oder $-(C_mH_{2m}O)_n-$ $R^{12}$, worin m 2 oder 3 ist, n eine Zahl von 2 bis 6 darstellt und $R^{12}$ H oder $C_1$-$C_{12}$-Alkyl bedeutet.

Die Gruppe $R^2$ ist bevorzugt ein Thiorest $R^{16}$-S-, worin $R^{16}$ unsubstituiertes oder wie zuvor angegeben substituiertes $C_1$-$C_{20}$-Alkyl, $C_2$-$C_{20}$-Alkenyl, $C_2$-$C_{20}$-Alkinyl, $C_3$-$C_8$-Cycloalkyl, $C_6$-$C_{12}$-Aryl oder $C_7$-$C_{12}$-Aralkyl darstellt oder $-(C_mH_{2m}O)_n-$ $R^{12}$ ist. In einer bevorzugten Ausführungsform stellen $R^2$, oder $R^2$ und $R^3$, oder $R^6$, oder $R^7$, oder $R^6$ und $R^7$, oder $R^2$ und $R^6$ oder $R^7$, oder $R^2$, $R^3$ und $R^6$ oder $R^7$, oder $R^2$, $R^3$, $R^6$ und $R^7$ einen Thiorest oder $-S-(C_mH_{2m}O)_n-$ $R^{12}$ dar. $R^1$, $R^4$, $R^5$ und $R^8$ sind insbesondere H.

$R^{16}$ enthält in der Bedeutung von Alkyl bevorzugt 1 bis 12, besonders 1 bis 8 und insbesondere 1 bis 4 C-Atome und es kann linear oder verzweigt sein. Beispiele sind zuvor erwähnt worden. $R^{16}$ als Alkenyl kann linear oder verzweigt sein und enthält bevorzugt 3 bis 12, besonders 3 bis 6 C-Atome und bevorzugt eine terminale Doppelbindung. Beispiele sind Allyl, Butyl-1-en-4-yl, But-2-en-4-yl, Pent-1-en-5-yl und Hex-1-en-6-yl. $R^{16}$ als Alkenyl kann linear oder verzweigt sein und enthält bevorzugt 3 bis 12, besonders 3 bis 6 C-Atome und bevorzugt eine terminale Dreifachbindung. Als Cycloalkyl enthält $R^{16}$ bevorzugt 4 bis 7, besonders 5 oder 6 Ring-C-Atome. Beispiel für Cycloalkyl sind Cyclopropyl, Cyclobutyl, Cyclopentyl, Cyclohexyl, Cycloheptyl und Cyclooctyl. $R^{16}$ als Aryl ist z.B. Naphthyl und besonders Phenyl, und als Aralkyl besonders Phenyl-$C_xH_{2x}$-, worin x eine Zahl von 1 bis 6, besonders 1 oder 2 ist. $R^{16}$ kann auch den Rest der Formel $-(C_mH_{2m}O)_n-$ $R^{12}$ bedeuten, worin $R^{12}$, m und n die zuvor angegebenen Bedeutungen, einschliesslich der Bevorzugungen haben.

Eine bevorzugte Ausführungsform sind solche Verbindungen der Formel I, worin $R^{16}$ für unsubstituiertes oder substituiertes $C_1$-$C_{20}$-Alkyl, Cyclopentyl, Cyclohexyl, Phenyl oder Benzyl oder $-(C_mH_{2m}O)_n-$ $R^{12}$ steht, wobei m eine Zahl von 2 bis 4, n eine Zahl von 2 bis 12 und $R^{12}$ H oder $C_1$-$C_{12}$-Alkyl bedeuten.

Eine andere bevorzugte Ausführungsform sind solche Verbindungen der Formel I, worin $R^{16}$ unsubstituiertes oder mit -OH, $-NR^{10}R^{11}$, $C_1$-$C_{18}$-Alkoxy, $-COOR^9$, oder $-CONR^{10}R^{11}$ substituiertes $C_1$-$C_{18}$-Alkyl oder $-(C_mH_{2m}O)_n-$ $R^{12}$ bedeutet, wobei m 2 oder 3 und n eine Zahl von 2 bis 6 sind und $R^{12}$ H oder $C_1$-$C_6$-Alkyl darstellt.

Eine bevorzugte Ausführungsform sind auch solche Verbindungen der Formel I, worin $R^{16}$ für unsubstituiertes $C_1$-$C_{18}$-Alkyl, oder mit $C_1$-$C_{12}$-Alkoxy oder $-COOR^9$, wobei $R^9$ $C_1$-$C_{12}$-Alkyl ist, substituiertes $C_1$ bis $C_6$-Alkyl, oder mit -OH oder $-NR^{10}R^{11}$, wobei $R^{10}$ und $R^{11}$ H oder $C_1$-$C_{12}$-Alkyl sind, substituiertes $C_2$-$C_6$-Alkyl steht.

Ganz besonders sind solche Verbindungen der Formel I bevorzugt, worin $R^{16}$ $C_1$-$C_{12}$-Alkyl, $C_6$-$C_{12}$-Aryl, $C_7$-$C_{18}$-Alkaryl, $-CH_2CH_2OH$, $-CH_2CHOHCH_2OH$, $-CH_2COOR^9$ oder $-CH_2CH_2COOR^9$ ist, und $R^9$ die zuvor angegebenen Bedeutungen hat, einschliesslich der Bevorzugungen. In diesem Fall stehen $R^1$ und $R^3$ bis $R^8$ besonders bevorzugt für H.

$R^1$ und $R^3$ bis $R^8$ bedeuten als Halogen vorzugsweise -F, -Cl oder -Br. $R^1$ und $R^3$ bis $R^8$ können unabhängig einen Rest der Formeln $-COOR^9$, $-NR^{10}R^{11}$ oder $-CONR^{10}R^{11}$ darstellen, wobei auch hier für $R^9$, $R^{10}$ und $R^{11}$ unabhängig die zuvor für diese Reste gegebenen Bevorzugungen gelten.

$R^1$ und $R^3$ bis $R^8$ können einen Rest der Formel $-SiR^{13}R^{14}R^{15}$ sein, worin $R^{13}$, $R^{14}$ und $R^{15}$ lineares oder verzweigtes Alkyl darstellen, das bevorzugt 1 bis 8, besonders 1 bis 4 C-Atome enthält. Einige Beispiele sind Trimethyl-, Ethyldimethyl-, Triethyl-, Tri-n- oder Tri-i-propyl-, n-Propyldimethyl-, Tri-n-butyl-, n-Butyldimethyl-, t-Butyldimethyl- und (1,1,2,2-Tetramethylethyl)dimethylsilyl.

$R^1$ und $R^3$ bis $R^8$ können unsubstituiertes oder substituiertes, lineares oder verzweigtes $C_1$-$C_{20}$-, bevorzugt $C_1$-$C_{18}$-, besonders $C_1$-$C_{12}$ und insbesondere $C_1$-$C_6$-Alkyl$(X)_p$ darstellen, worin p 0 oder 1 und X O, S, SO oder $SO_2$, besonders O oder S sind. Einige Beispiele sind Methyl, Ethyl und die Isomeren von Propyl, Butyl, Pentyl, Hexyl, Heptyl, Octyl, Nonyl, Decyl, Undecyl, Dodecyl, Tetradecyl, Hexadecyl, Octadecyl, Eicosyl und entsprechende Alkoxy-, Alkylthio-, Alkylsulfinyl- und Alkylsulfonylreste, 2-Hydroxyethyloxy oder -thio, 1,2-Dihydroxypropyloxy, 2-Aminoethyloxy oder -thio, 4-Hydroxylbutyloxy, 6-Hydroxyhexyloxy, 10-Hydroxydecyloxy, Di(methoxycarbonyl)methyl, 1,1-Di(methoxycarbonyl)ethyl, Cyano(methoxycarbonyl)methyl, Carboxymethyl, Trifluormethyl, Carbamoylmethyl, (Ethoxycarbonyl)-methoxy.

$R^1$ und $R^3$ bis $R^8$ können unsubstituiertes oder substituiertes $C_2$-$C_{18}$-, bevorzugt $C_2$-$C_{12}$- und besonders $C_2$-$C_6$-Alkenyl$(X)_p$ - oder -Alkinyl$(X)_p$ sein, das linear oder verzweigt sein kann, worin p 0 oder 1 und X O, S, SO oder $SO_2$, bevorzugt S oder O bedeuten. Wenn p für 1 steht, ist die Alkenyl-bzw. Alkinylgruppe vorzugsweise mindestens eine $CH_2$-Gruppe von den Benzolringen in den Verbindungen der Formel I getrennt. Einige

Beispiele sind Vinyl, Ethinyl sowie Allyl, Propargyl, Crotonyl, 1-Buten-3- oder 4-yl, 1- oder 2-Penten-5-yl, 1-, 2- oder 3-Hexen-6-yl, 1-Hepten-7-yl, 1-Octen-8-yl, 1-Nonen-9-yl, 1-Decen-10-yl, 1-Undecen-11-yl, 1-Dodecen-12-yl, und entsprechende Alken- und Alkinoxy, -thio, -sulfinyl und sulfonylreste.

$R^1$ und $R^3$ bis $R^8$ können unsubstituiertes oder substituiertes $C_3$-$C_8$-, bevorzugt $C_4$-$C_7$ und besonders $C_5$- oder $C_6$-Cycloalkyl$(X)_p$ darstellen, worin p 0 oder 1 ist und X S, O, SO oder $SO_2$ bedeutet. X steht bevorzugt für O oder S. Beispiele sind Cyclopentyl, Cyclohexyl, Methylcyclohexyl, Cyclohexyloxy und Cyclohexylthio.

$R^1$ und $R^3$ bis $R^8$ können gegebenenfalls substituiertes $C_6$-$C_{12}$-Aryl$(X)_p$ oder $C_6$-$C_{12}$-Aralkyl$(X)_p$ sein, worin X für O, S, SO oder $SO_2$, besonders für O oder S, und p für 0 oder 1 stehen, und das Aryl bevorzugt Naphthyl und besonders Phenyl ist. Das Aralkyl entspricht bevorzugt Phenyl$(C_zH_{2z})$ mit z in der Bedeutung einer Zahl von 1 bis 6, besonders 1 bis 4 und ist insbesondere Benzyl. Beispiele sind Phenyl, Benzyl, Methylphenyl, Ethylphenyl, Dodecylphenyl, Methylbenzyl, Phenylthio, Phenoxy, Chlorphenoxy, Methoxyphenoxy, Methylthiophenoxy, Methoxyphenylthio, Benzylthio, Methoxybenzylthio, Methylbenzylthio.

Bei $R^1$ und $R^3$ bis $R^8$ kann es sich um den Rest der Formel $-Y(C_mH_{2m}O)_n R^{12}$ handeln, worin Y O oder S bedeuten, und m, n und $R^{12}$ die zuvor für die Gruppe $(C_mH_{2m}O)_n R^{12}$ angegebenen Bedeutungen haben, einschliesslich der Bevorzugungen. Die $C_mH_{2m}$-Gruppe ist besonders $-CH_2CH_2-$, $-CH_2CH_2CH_2-$ oder $-CH_2CHCH_3-$. Einige Beispiele sind $-O(CH_2CH_2O)_n R^{12}$, $-S(CH_2CH_2O)_n R^{12}$, $-O(CH_2CHCH_3O)_n R^{12}$ und $-S(CH_2CHCH_3O)_n R^{12}$, worin n für 2, 3, 4, 5, 6, 7, 8, 9, 10, 11 oder 12 und $R^{12}$ für H, Methyl, Ethyl, n- oder i-Propyl, n-, i- oder t-Butyl, Pentyl oder Hexyl stehen.

$R^2$, $R^3$, und $R^5$ bis $R^8$, besonders $R^2$, $R^3$, $R^6$ und/oder $R^7$ bedeuten als Thiorest bevorzugt $C_1$-$C_{12}$-Alkylthio, Phenylthio, Benzylthio, $C_1$-$C_4$-Alkylphenylthio- oder -benzylthio, $C_1$-$C_4$-Alkoxyphenylthio- oder -benzylthio, Halogen-, besonders Fluor-, Chlor-oder Bromphenylthio- oder benzylthio, $-SCH_2CH_2OH$, $-SCH_2CHOHCH_2OH$, $-SCH_2COOR^9$ und $-SCH_2CH_2COOR^9$ mit $R^9$ in der zuvor angegebenen Bedeutung, oder $-S(C_mH_{2m}O)_n R^{12}$ worin m 2 oder 3, n 2 bis 20 und $R^{12}$ H oder $C_1$-$C_{12}$-Alkyl bedeuten.

Eine bevorzugte Ausführungsform sind solche Verbindungen der Formel I, worin $R^1$ und $R^3$ bis $R^8$ unabhängig voneinander H, -CN, $-NO_2$, -F, -Cl, -Br, $-COOR^9$, $-NR^{10}R^{11}$, $-CONR^{10}R^{11}$, wobei $R^9$ H oder $C_1$-$C_{12}$-Alkyl und $R^{10}$ und $R^{11}$ unabhängig voneinander H, $C_1$-$C_{12}$-Alkyl, $-CH_2CH_2OH$, $-CH_2CH(OH)CH_3$, $-CH_2CHOHCH_2OH$ oder $(C_mH_{2m}O)_n R^{12}$ mit m gleich 2 oder 3, n gleich einer Zahl von 2 bis 12 und $R^{12}$ mit der Bedeutung von H oder $C_1$-$C_{12}$-Alkyl, oder $-SiR^{13}R^{14}R^{15}$ mit $R^{13}$, $R^{14}$ und $R^{15}$ in der Bedeutung von $C_1$-$C_4$-Alkyl, oder unsubstituiertes oder substituiertes $C_1$-$C_{18}$-Alkyl$(X)_p$, $C_6H_5(X)_p$ oder $C_6H_5CH_2(X)_p$, wobei X O oder S und p 0 oder 1 bedeuten, oder $-Y(C_mH_{2m}O)_n R^{12}$, wobei Y O oder S, m 2 oder 3, n eine Zahl von 2 bis 12 und $R^{12}$ H oder $C_1$-$C_{12}$-Alkyl sind, darstellen.

In einer anderen bevorzugten Ausführungsform stellt $R^1$ H, $-CF_3$, -OH, $C_1$-$C_{18}$-Alkyl, unsubstituiertes oder mit -OH substituiertes $C_1$-$C_{18}$-Alkoxy, unsubstituiertes oder mit Halogen, $C_1$-$C_6$-Alkyl oder -Alkoxy substituiertes $C_6$-$C_{10}$-Aryloxy, $C_7$-$C_{10}$-Aralkyloxy oder $-COOR^9$ mit $R^9$ in der Bedeutung eines um das Hydroxylwasserstoffatom verminderten $C_1$-$C_{20}$-Alkohols dar. $R^1$ steht besonders für H.

In einer weiteren bevorzugten Ausführungsform stehen $R^4$, $R^5$ und $R^8$ für H. Besonders stehen $R^1$, $R^4$, $R^5$ und $R^8$ für H.

Bevorzugt sind auch Verbindungen der Formel I, worin $R^3$, oder $R^3$ und $R^6$ oder $R^7$, oder $R^3$, $R^6$ und $R^7$ die Bedeutung $R^{16}S$- haben; oder worin $R^1$, $R^4$, $R^5$ und $R^8$ für H stehen, $R^2$, $R^3$ oder beide $-SR^{16}$ und $R^6$ und $R^7$ H, oder $R^2$, $R^6$ oder $R^7$ $-SR^{16}$ und $R^3$ und $R^7$ oder $R^3$ und $R^6$ H bedeuten; oder worin $R^1$ und $R^3$ bis $R^8$ für H stehen und $R^{16}$ die zuvor angegebene Bedeutung hat.

Ein weiterer Gegenstand der Erfindung ist ein Verfahren zur Herstellung von Verbindungen der Formel I, das dadurch gekennzeichnet ist, dass man in einer Diels-Alder-Reaktion eine Verbindung der Formel II

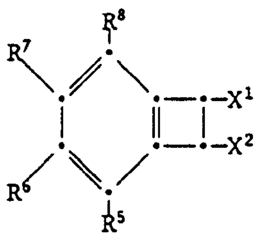

(II),

worin $R^5$, $R^6$, $R^7$ und $R^8$ die zuvor angegebenen Bedeutungen haben und $X^1$ und $X^2$ unabhängig voneinander für -Cl, -Br - oder -J stehen, unter Abspaltung von $HX^1$ und $HX^2$ mit einer Verbindung der Formel III

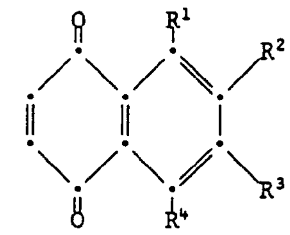

(III),

worin $R^1$, $R^2$, $R^3$ und $R^4$ die zuvor angegebenen Bedeutungen haben, umsetzt.

Die Reaktion wird vorteilhaft bei Temperaturen von 50 bis 250°C, bevorzugt 80 bis 200°C durchgeführt. Zweckmässig wird ein inertes Lösungsmittel verwendet, zum Beispiel polare aprotische Lösungsmittel. Einige Beispiele sind aromatische Kohlenwasserstoffe (Benzol, Toluol, Xylol, Chlorbenzol und Dichlorbenzol), Nitrile (Acetonitril), Ether (Dibutylether, Dioxan, Tetrahydrofuran, Ethylenglykol- oder Diethylenglykoldimethylether). Die Isolierung und Reinigung kann nach üblichen Methoden erfolgen, z.B. Kristallisation, Destillation oder Chromatographie.

Verbindungen der Formel II sind teilweise bekannt (siehe z.B. H.P. Cava et al., J. Am. Chem. Soc., S. 1701 (1957) und J.W. Barton et al., J. Chem. Soc. Perkin Trans. 1, S. 967-971 (1986)), bzw. nach analogen Verfahren herstellbar. Die für die Herstellung benötigten substituierten 1,2-Bis(dichlor- oder -dibrommethyl)benzole sind ebenfalls teilweise bekannt oder durch übliche elektrophile oder nukleophile Substitutionsreaktionen entsprechender o-Dimethylbenzole und deren anschliessenden Chlorierung oder Bromierung mit z.B. N-Chlor- oder N-Bromsuccinimid erhältlich.

Die p-Naphthochinone der Formel III sind neu und z.B. durch nukleophile Substitution von gegebenenfalls geschützten und substituierten 6-Halogen- oder 6-Nitro-1,4-naphthochinonen mit $R^2SH$ in Gegenwart von Alkalimetallverbindungen ($K_2CO_3$, $Cs_2CO_3$, KOH, NaOH, $NaNH_2$, $NaOCH_3$, $NaOC_2H_5$) oder mit $R^2SX^1$, worin $X^1$ z.B. Li, K, Na, Rb oder Cs steht, erhältlich. Halogen- und Nitro-naphthochinone sind z.B. in Houben-Weyl, Chinone I, Band 7/3b (1977) beschrieben. Die Naphthochinone der Formel III können auch in bekannter Weise durch elektrophile oder nukleophile Substitution von gegebenenfalls substituierten Naphthalinen, Dihydro- oder Tetrahydronaphthalinen und anschliessende Umwandlung in die substituierten 1,4-Naphthochinone hergestellt werden.

Die Verbindungen der Formel I können auch hergestellt werden, indem man 1,2-Bis(dihalogenmethyl)benzole der Formel

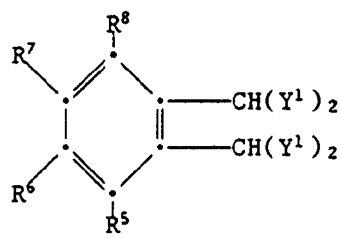

,

worin $Y^1$ für Cl, Br oder I steht, in Gegenwart von NaI in einem organischen Lösungsmittel mit einer Verbindung der Formel III umsetzt. Diese Methode ist von J.W. McOmie in Synthesis, S. 416-417 (1973) beschrieben.

Verbindungen der Formel I können auch erhalten werden, indem man Anthracen-1,4-chinone der Formel

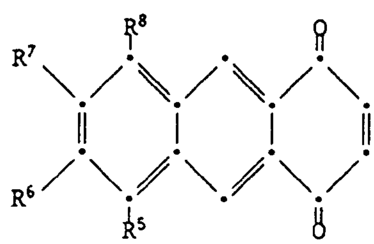

mit einem α-Pyron der Formel IV

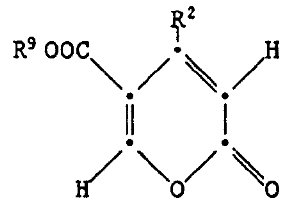

(IV),

oder einem Butadien der Formel V

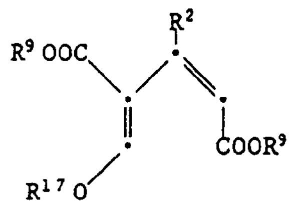

(V)

umsetzt, wobei $R^{17}$ $C_1$-$C_6$-Alkyl ist, $R^9$ die zuvor angegebene Bedeutung hat und bevorzugt $C_1$-$C_6$-Alkyl ist. Diese Methode und die Herstellung von α-Pyronen ist in der US-PS 4,617,151 und der EP-A-0 195 743 beschrieben.

Verbindungen der Formeln IV und V sind z.B. folgendermassen erhältlich:

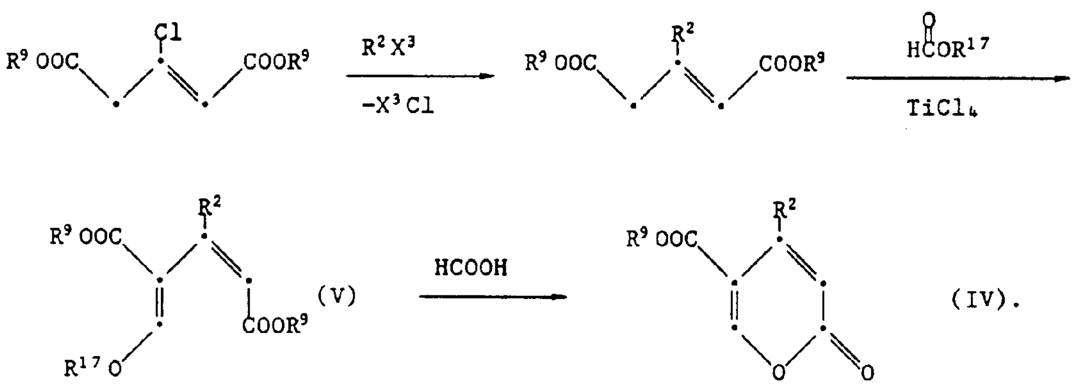


Die Verbindungen der Formel I können in bekannter Weise auch erhalten werden, indem man gegebenenfalls substituierte Naphthalindicarbonsäureanhydride der Formel

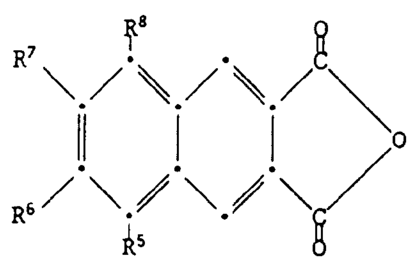

nach Friedel-Crafts mit einem Benzol der Formel

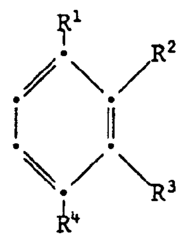

,

in Gegenwart einer Lewissäure cyclisiert.

Nach den beschriebenen Verfahren können auch Naphthacen-5,12-dione mit nukleophil substituierbaren Gruppen (z.B. $-NO_2$, F, Cl, Br) in den 1-, 2-, 3-, 4-, 7-, 8-, 9- und/oder 10-Stellungen hergestellt werden, in denen durch nucleophile Substituenten andere nucleophile Gruppen $R^1$ bis $R^8$ und $R^{16}S$- eingeführt werden können. Wenn $R^{16}$ einen Polyoxaalkylenrest bedeutet, so erhält man solche Verbindungen auch durch Umsetzung von Verbindungen der Formel I mit $R^{16}$ gleich Hydroxyalkyl mit Epoxiden. Ferner ist es möglich die Reste $R^1$ bis $R^8$ durch klassische Reaktionen abzuwandeln, wie z.B. Hydrolyse, Ver- oder Umesterung, Amidierung, Oxidation oder Reduktion.

Die Verbindungen der Formel I sind im allgemeinen kristallin und zeichnen sich durch eine hohe thermische Stabilität aus. Die Verbindungen können sehr gut in härtbaren Zusammensetzungen gelöst werden, gegebenenfalls zusammen mit einem Lösungsmittel.

Sie eignen sich alleine oder zusammen mit H-Donoren wie tertiären Aminen, Alkoholen oder Phenylessigsäurederivaten hervorragend als sehr wirksame Photoinitiatoren bzw. Sensibilisatoren für die lichtinduzierte Polymerisation bzw. Dimerisation ethylenisch ungesättigter Verbindungen. Sie zeichnen sich hierbei durch eine hohe Lichtempfindlichkeit und Wirksamkeit über einen Wellenlängenbereich von ca. 200 nm (UV-Licht) bis etwa 600 nm aus. Die Eigenschaften der erfindungsgemässen Verbindungen, z.B. Löslichkeit, Schmelzpunkt und Absorptionsbereich lassen sich durch die Wahl von Substituenten gezielt beeinflussen.

Ein weiterer Gegenstand vorliegender Erfindung ist eine durch Strahlung polymerisierbare Zusammensetzung, enthaltend (a) mindestens eine nichtflüchtige, monomere, oligomere oder polymere Verbindung mit mindestens einer polymerisierbaren oder dimerisierbaren ethylenisch ungesättigten Doppelbindung und (b) mindestens eine Verbindung der Formel I als Photoinitiator oder Sensibilisator.

Die Zusammensetzungen können weitere von (b) verschiedene Photoinitiatoren oder Sensibilisatoren enthalten.

Die Zusatzmenge an erfindungsgemässen Verbindungen richtet sich im wesentlichen nach wirtschaftlichen Gesichtspunkten, deren Löslichkeiten und nach der gewünschten Empfindlichkeit. Im allgemeinen werden 0,01 bis 20, vorzugsweise 0,05-10 und besonders 0,1 bis 5 Gew.% verwendet, bezogen auf die Komponente (a).

Als Komponente (a) kommen solche ethylenisch ungesättigten monomeren, oligomeren und polymeren Verbindungen in Frage, die durch Photopolymerisation zu höhermolekularen Produkten reagieren und hierbei ihre Löslichkeit verändern.

Besonders geeignet sind z.B. Ester von ethylenisch ungesättigten Carbonsäuren und Polyolen oder Polyepoxiden, und Polymere mit ethylenisch ungesättigten Gruppen in der Kette oder in Seitengruppen, wie z.B. ungesättigte Polyester, Polyamide und Polyurethane und Copolymere hiervon, Polybutadien und Butadien-Copolymere, Polyisopren und Isopren-Copolymere, Polymere und Copolymere mit (Meth)Acrylgruppen in Seitenketten, sowie Mischungen von einem oder mehreren solcher Polymerer.

Beispiele für ungesättigte Carbonsäuren sind Acrylsäure, Methacrylsäure, Crotonsäure, Itaconsäure, Zimtsäure, ungesättigte Fettsäuren wie Linolensäure oder Oelsäure. Bevorzugt sind Acryl- und Methacrylsäure.

Als Polyole sind aromatische und besonders aliphatische und cycloaliphatische Polyole geeignet. Beispiele für aromatische Polyole sind Hydrochinon, 4,4'-Dihydroxydiphenyl, 2,2-Di(4-hydroxyphenyl)-propan, sowie Novolake und Resole. Beispiele für Polyepoxide sind solche auf der Basis der genannten Polyole, besonders der aromatischen Polyole und Epichlorhydrin. Ferner sind auch Polymere oder Copolymere, die Hydroxylgruppen in der Polymerkette oder in Seitengruppen enthalten, wie z.B. Polyvinylalkohol und Copolymere davon oder Polymethacrylsäurehydroxyalkylester oder Copolymere davon, als Polyole geeignet. Weitere geeignete Polyole sind Oligoester mit Hydroxylendgruppen.

Beispiele für aliphatische und cycloaliphatische Polyole sind Alkylendiole mit bevorzugt 2 bis 12 C-Atomen, wie Ethylenglykol, 1,2- oder 1,3-Propandiol, 1,2-, 1,3 oder 1,4-Butandiol, Pentandiol, Hexandiol, Octandiol, Dodecandiol, Diethylenglykol, Triethylenglykol, Polyethylenglykole mit Molekulargewichten von bevorzugt 200 bis 1500, 1,3-Cyclopentandiol, 1,2-, 1,3- oder 1,4-Cyclohexandiol, 1,4-Dihydroxymethylcyclohexan, Glycerin, Tris-(ß-hydroxyethyl)amin, Trimethylolethan, Trimethylolpropan, Pentaerythrit, Dipentaerythrit und Sorbit.

Die Polyole können teilweise oder vollständig mit einer oder verschiedenen ungesättigten Carbonsäuren verestert sein, wobei in Teilestern die freien Hydroxylgruppen modifiziert, z.B. verethert oder mit anderen Carbonsäuren verestert sein können.

Beispiele für Ester sind:
Trimethylolpropantriacrylat, Trimethylolethantriacrylat, Trimethylolpropantrimethacrylat, Trimethylolethantrimethacrylat, Tetramethylenglykoldimethacrylat, Triethylenglykoldimethacrylat, Tetraethylenglykoldiacrylat, Pentaerythritdiacrylat, Pentaerythrittriacrylat, Pentaerythrittetraacrylat, Dipentaerythritdiacrylat, Dipentaerythrittriacrylat, Dipentaerythrittetraacrylat, Dipentaerythritpentaacrylat, Dipentaerythrithexaacrylat, Tripentaerythritoctaacrylat, Pentaerythritdimethacrylat, Pentaerythrittrimethacrylat, Dipentaerythritdimethacrylat, Dipentaerythrittetramethacrylat, Tripentaerythritoctamethacrylat, Pentaerythritdiitaconat, Dipentaerythrittrisitaconat, Dipentaerythritpentaitaconat, Dipentaerythrithexaitaconat, Ethylenglykoldimethacrylat, 1,3-Butandioldiacrylat, 1,3-Butandioldimethacrylat, 1,4-Butandioldiitaconat, Sorbittriacrylat, Sorbittetraacrylat, Pentaerythrit-modifiziert-triacrylat, Sorbittetramethacrylat, Sorbitpentaacrylat, Sorbithexaacrylat, Oligoesteracrylate und -methacrylate, Glyzerindi- und -triacrylat, 1,4-Cyclohexandiacrylat, Bisacrylate und Bismethacrylate von Polyethylenglykol mit Molekulargewicht von 200-1500, oder Gemische davon.

Als Komponente (a) sind auch die Amide gleicher oder verschiedener ungesättigter Carbonsäuren von aromatischen, cycloaliphatischen und aliphatischen Polyaminen mit bevorzugt 2 bis 6, besonders 2 bis 4 Aminogruppen geeignet. Beispiele für solche Polyamine sind Ethylendiamin, 1,2oder 1,3-Propylendiamin, 1,2-, 1,3- oder 1,4-Butylendiamin, 1,5-Pentylendiamin, 1,6-Hexylendiamin, Octylendiamin, Dodecylendiamin, 1,4-Diaminocyclohexan, Isophorondiamin, Phenylendiamin, Bisphenylendiamin, Di-ß-aminoethylether, Diethylentriamin, Triethylentetramin, Di-(ß-aminoethoxy)- oder Di(ß-aminopropoxy)ethan. Weitere geeignete Polyamine sind Polymere und Copolymere mit Aminogruppen in der Seitenkette und Oligoamide mit Aminoendgruppen.

Beispiele für solche ungesättigten Amide sind: Methylen-bis-acrylamid, 1,6-Hexamethylen-bis-acrylamid, Diethylentriamin-tris-methacrylamid, Bis(methacrylamidopropoxy)-ethan, ß-Methacrylamidoethylmethacrylat, N[(ß-Hydroxyethoxy)ethyl]-acrylamid.

Geeignete ungesättigte Polyester und Polyamide leiten sich z.B. von Maleinsäure und Diolen oder Diaminen ab. Die Maleinsäure kann teilweise durch andere Dicarbonsäuren ersetzt sein. Sie können zusammen mit ethylenisch ungesättigten Comonomeren, z.B. Styrol, eingesetzt werden. Die Polyester und Polyamide können sich auch von Dicarbonsäuren und ethylenisch ungesättigten Diolen oder Diaminen ableiten, besonders von längerkettigen mit z.B. 6 bis 20 C-Atomen. Beispiele für Polyurethane sind solche, die aus gesättigten oder ungesättigten Diisocyanaten und ungesättigten bzw. gesättigten Diolen aufgebaut sind.

Polybutadien und Polyisopren und Copolymere davon sind bekannt. Geeignete Comonomere sind z.B. Polyolefine wie Ethylen, Propen, Buten, Hexen, (Meth)Acrylate, Acrylnitril, Styrol oder Vinylchlorid. Polymere mit (Meth)Acrylatgruppen in der Seitenkette sind ebenfalls bekannt. Es kann sich z.B. um Umsetzungsprodukte von Epoxidharzen auf Novolakbasis mit (Meth)Acrylsäure handeln, um Homo- oder Copolymere des Polyvinylalkohols oder deren Hydroxyalkylderivaten, die mit (Meth)Acrylsäure verestert sind, oder um Homo- und Copolymere von (Meth)Acrylaten, die mit Hydroxyalkyl(meth)acrylaten verestert sind.

Die photopolymerisierbaren Verbindungen können alleine oder in beliebigen Mischungen eingesetzt werden. Bevorzugt werden Gemische von Polyol(Meth)Acrylaten verwendet.

Geeignete dimerisierbare Verbindungen sind solche, die z.B. Zimtsäurereste, 3,4-substituierte Maleinimidylreste oder

$$-CH{=}CH-\overset{\overset{O}{\|}}{C}-$$

oder

$$-CH{=}CH-\overset{\overset{O}{\|}}{C}-CH{=}CH\text{-Reste}$$

enthalten.

Diese Reste sind im allgemeinen an Oligomere oder Polymere gebunden, z.B. in der Polymerkette oder als Seitengruppen. Polymere mit Maleinimidylgruppen sind z.B. in der DE-A-2626795 beschrieben. Die Maleinimidylgruppe kann der Formel

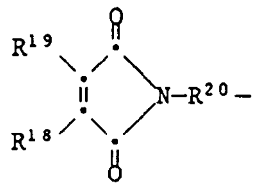

entsprechen, worin $R^{18}$ und $R^{19}$ $C_1$-$C_{12}$-Alkyl, $C_5$- oder $C_6$-Cycloalkyl, $C_6$-$C_{12}$-Aryl, Halogen (z.B. Cl oder Br), oder $R^{18}$ und $R^{19}$ zusammen Tri- oder Tetramethylen bedeuten, und $R^{20}$ eine Brückengruppe, z.B. $C_2$-$C_{12}$-Alkylen oder Phenylen, darstellt.
Epoxidharze mit

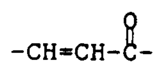

oder

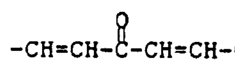Gruppen

sind
z.B. in der DE-A-2342407 beschrieben.

Den erfindungsgemässen Zusammensetzungen können auch Bindemittel zugesetzt werden, was besonders zweckmässig ist, wenn es sich bei den photopolymerisierbaren bzw. photodimerisierbaren Verbindungen um flüssige oder viskose Substanzen handelt. Die Menge des Bindemittels kann z.B. 5-95, vorzugsweise 10-90 und besonders 50-90 Gew.% betragen, bezogen auf die gesamte Zusammensetzung. Die Wahl des Bindemittels erfolgt je nach dem Anwendungsgebiet und hierfür geforderter Eigenschaften wie Entwickelbarkeit in wässrigen und organischen Lösungsmittelsystemen, Adhäsion auf Substraten und Sauerstoffempfindlichkeit.

Geeignete Bindemittel sind z.B. Polymere mit einem Molekulargewicht von etwa 5000-2 000 000, bevorzugt 10 000 bis 1 000 000. Beispiele sind: Homo- und copolymere Acrylate und Methacrylate, z.B. Copolymere aus Methylmethacrylat/Ethylacrylat/Methacrylsäure, Poly(methacrylsäurealkylester), Poly(acrylsäurealkylester); Celluloseester und -ether wie Celluloseacetat, Celluloseacetatbutyrat, Methylcellulose, Ethylcellulose; Polyvinylbutyral, Polyvinylformal, cyclisierter Kautschuk, Polyether wie Polyethylenoxid, Polypropylenoxid, Polytetrahydrofuran; Polystyrol, Polycarbonat, Polyurethan, chlorierte Polyolefine, Polyvinylchlorid, Copolymere aus Vinylchlorid/Vinylidenchlorid, Copolymere von Vinylidenchlorid mit Acrylnitril, Methylmethacrylat und Vinylacetat, Polyvinylacetat, Copoly(ethylen/vinylacetat), Polyamide wie Polycaprolactam und Poly(hexamethylenadipamid), Polyester wie Poly(äthylenglykolterephthalat) und Poly(hexamethylenglykolsuccinat).

Die erfindungsgemässen Zusammensetzungen eignen sich als Beschichtungsmittel für Substrate aller Art, z.B. Holz, Papier, Keramik, Kunststoffe, wie Polyester und Celluloseacetatfilme, und Metalle, wie Kupfer und Aluminium, bei denen durch Photopolymerisation eine Schutzschicht oder eine photographische Abbildung aufgebracht werden soll. Ein weiterer Gegenstand vorliegender Erfindung sind die beschichteten Substrate und ein Verfahren zum Aufbringen photographischer Abbildungen auf den Substraten. Die beschichteten Substrate können auch als Aufzeichnungsmaterial für Hologramme (Volumen-Phasen-Diagramm) verwendet werden, wobei vorteilhaft ist, dass für diesen Zweck keine Nassentwicklung notwendig ist.

Die Beschichtung der Substrate kann erfolgen, indem man eine flüssige Zusammensetzung, eine Lösung oder Suspension auf das Substrat aufbringt. Flüssige Zusammensetzungen ohne Lösungsmittel sind bevorzugt.

Die Wahl des Lösungsmittels und die Konzentration richtet sich hauptsächlich nach der Art der Zusammensetzung und nach dem Beschichtungsverfahren. Die Zusammensetzung wird mittels bekannter Beschichtungsverfahren auf ein Substrat gleichförmig aufgebracht, z.B. durch Tauchen, Rakelbeschichtung, Vorhanggiessverfahren, Elektrophorese, Aufpinseln, Sprayen oder Reverseroll-Beschichtung. Die Auftragsmenge (Schichtdicke) und Art des Substrates (Schichtträger) sind abhängig vom gewünschten Applikationsgebiet. Als Schichtträger für photographische Informationsaufzeichnung dienen z.B. Folien aus Polyester, Celluloseacetat oder mit Kunststoff beschichtete Papiere; für Offsetdruckformen speziell behandeltes Aluminium und für die Herstellung gedruckter Schaltungen kupferkaschierte Laminate. Die Schichtdicken für photographische Materialien und Offsetdruckformen betragen im allgemeinen ca. 0,5 bis ca. 10 µm; für gedruckte Schaltungen im allgemeinen 1 bis ca. 100 µm. Bei Mitverwendung von Lösungsmitteln werden diese nach dem Beschichten entfernt.

Photohärtbare Zusammensetzungen, wie sie für die verschiedenen Zwecke verwendet werden, enthalten meist ausser den photopolymerisierbaren Verbindungen und den Photoinitiatoren eine Reihe sonstiger Zusätze. So ist es vielfach üblich, thermische Inhibitoren zuzusetzen, die vor allem während der Herstellung der Zusammensetzungen durch Mischen der Komponenten vor einer vorzeitigen Polymerisation schützen sollen. Hierzu werden beispielsweise Hydrochinon, Hydrochinonderivate, p-Methoxyphenol, ß-Naphthole oder sterisch gehinderte Phenole wie z.B. 2,6-Di(tert-butyl)-p-kresol verwendet. Weiter können geringe Mengen von UV-Absorbern zugesetzt werden, wie z.B. solche vom Benztriazol-, Benzophenon- oder Oxalanilid-Typ. Ebenso lassen sich Lichtschutzmittel vom Typus sterisch gehinderter Amine (HALS) zusetzen.

Zur Erhöhung der Dunkellagerstabilität können Kupferverbindungen, wie Kupfernaphthenat, -stearat, oder -octoat, Phosphorverbindungen, wie Triphenylphosphin, Tributylphosphin, Triethylphosphit, Triphenylphosphit oder Tribenzylphosphit, quaternäre Ammoniumverbindungen, wie Tetramethylammoniumchlorid oder Trimethyl-benzylammoniumchlorid oder Hydroxylaminderivate, wie z.B. N-Diethylhydroxylamin, zugesetzt werden.

Um die inhibierende Wirkung des Luftsauerstoffs auszuschliessen, setzt man photohärtbaren Gemischen häufig Paraffin oder ähnliche wachsartige Stoffe zu. Diese schwimmen bei Beginn der Polymerisation wegen mangelnder Löslichkeit im Polymeren aus und bilden eine transparente Oberflächenschicht, die den Zutritt von Luft verhindert.

Weitere übliche Zusätze sind Photosensibilisatoren, welche in bestimmten Wellenlängen absorbieren und die absorbierte Energie an den Initiatoren weitergeben oder selbst als zusätzlicher Initiator fungieren. Beispiele hierfür sind vor allem Thioxanthon-, Anthracen-, Anthrachinon- und Cumarinderivate.

Weitere übliche Zusätze sind Beschleuniger vom Amin-Typ, die vor allem in pigmentierten Zubereitungen von Bedeutung sind, da sie als Kettenüberträger wirken. Beispiele hierfür sind N-Methyldiethanolamin, Triethylamin, p-Dimethylaminobenzoesäureethylester oder Michler's Keton. Die Wirkung der Amine kann verstärkt werden durch den Zusatz von aromatischen Ketonen vom Benzophenontyp.

Weitere übliche Zusätze sind z.B. Füllstoffe, Pigmente, Farbstoffe, Haft-, Netz- und Verlaufmittel.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Insbesondere für den Siebdruck sind UV-härtbare Druckfarben von Bedeutung.

Gut geeignet sind die erfindungsgemässen photohärtbaren Zusammensetzungen auch zur Herstellung von Druckplatten, insbesondere Flexodruckplatten. Hierbei werden z.B. Gemische von löslichen linearen Polyamiden oder von Styrol-Butadien-Kautschuk mit photopolymerisierbaren Monomeren, beispielsweise Acrylamiden oder Acrylaten, und einem Photoinitiator verwendet. Filme und Platten aus diesen Systemen werden über das Negativ (oder Positiv) der Druckvorlage belichtet und die ungehärteten Anteile anschliessend mit einem Lösungsmittel eluiert.

Ein weiteres Einsatzgebiet der Photohärtung ist die Metallbeschichtung, beispielsweise bei der Lackierung von Blechen für Tuben, Dosen oder Flaschenverschlüsse, sowie die Photohärtung von Kunststoffbeschichtungen, beispielsweise von Fussboden- oder Wandbelägen auf PVC-Basis.

Beispiele für die Photohärtung von Papierbeschichtungen sind die farblose Lackierung von Etiketten, Schallplatten-Hüllen oder Buchumschlägen.

Wichtig ist auch die Verwendung der photohärtbaren Zusammensetzungen für Abbildungsverfahren und zur optischen Herstellung von Informationsträgern. Hierbei wird die auf dem Träger aufgebrachte Schicht (nass oder trocken) durch eine Photomaske mit kurzwelligem Licht bestrahlt und die unbelichteten Stellen der Schicht durch Behandlung mit einem Lösungsmittel (= Entwickler) entfernt. Die belichteten Stellen sind vernetztpolymer und dadurch unlöslich und bleiben auf dem Träger stehen. Bei entsprechender Anfärbung entstehen sichtbare Bilder. Ist der Träger eine metallisierte Schicht, so kann das Metall nach dem Belichten und Entwickeln an den unbelichteten Stellen weggeätzt oder durch Galvanisieren verstärkt werden. Auf diese Weise lassen sich gedruckte Schaltungen herstellen.

Zur Belichtung eignen sich Lichtquellen mit hohem Anteil an kurzwelligem Licht. Hierfür stehen heute entsprechende technische Vorrichtungen und verschiedene Lampenarten zur Verfügung. Beispiele sind Kohlelichtbogenlampen, Xenonlichtbogenlampen, Quecksilberdampflampen, Metall-Halogenlampen, Fluoreszenzlampen, Argonlampen oder photographische Flutlichtlampen. Neuerdings werden auch Laserlichtquellen verwendet. Diese haben den Vorteil, dass keine Photomasken notwendig sind; der gesteuerte Laserstrahl schreibt direkt auf die photohärtbare Schicht.

Weitere Gegenstände der Erfindung sind somit

a) ein beschichtetes Substrat, das auf mindestens einer Oberfläche mit einer erfindungsgemässen Zusammensetzung beschichtet ist;

b) ein Verfahren zur photographischen Herstellung von Reliefabbildungen oder Beschichtungen, dadurch gekennzeichnet, dass man ein beschichtetes Substrat bildmässig oder flächenmässig belichtet und unbelichtete Anteile danach mit einem Lösungsmittel entfernt;

c) die Verwendung von Verbindungen der Formel I als Initiatoren und Sensibilisatoren für die Photopolymerisation oder Photodimerisation von nichtflüchtigen monomeren, oligomeren oder polymeren Verbindungen mit mindestens einer polymerisierbaren oder dimerisierbaren ethylenisch ungesättigten Doppelbindung; und

d) die Verwendung einer erfindungsgemässen Zusammensetzung zur Herstellung von Lacken, Druckfarben, Druckplatten, Resistmaterialien sowie als Bildaufzeichnungsmaterial und Beschichtungsmittel.

Die erfindungsgemässen Photoinitiatoren können auch bei der Herstellung von Polymeren verwendet werden, z.B. für Flockungsmittel oder Hygieneartikel (siehe z.B. EP-A-0,031,005 und DE-A-2,545,290).

Die Verbindungen der Formel I sind wertvolle Zwischenprodukte zur Herstellung von substituierten Tetrathio- bzw. Tetraselenotetracenen (vgl. US-PS 4 617 151). Aus solchen chalkogenierten Tetracenen können mit Elektronenacceptoren elektrisch leitende Charge-Transferkomplexe (CT-Komplexe) hergestellt werden. Mit deren funktionellen Substituenten kann man sie an Polymere binden, z.B. als Seitengruppen in Polymere einbauen (vgl. US-PS 4,617,151). Die CT-Komplexe eignen sich auch zur Herstellung z.B. von antistatischen Beschichtungen photographischer Filmelemente, Magnetbänder, elektrophotographischer Filmelemente und elektronischer Komponenten (siehe US-PS 3,634,336). Die chalkogenierten Tetracene weisen ferner elektrochrome Eigenschaften auf; sie können für elektrochrome Displays verwendet werden. Sie eignen sich auch als laser-optische Datenspeicher [Nach. Chem. Techn. Lab. 35, S. 255 ff (1987)] und als Anodenmaterial in organischen Solid-State-Batterien (EP-A-0 090 598). CT-Komplexe von substituierten Tetrathio- oder Tetraselenotetracenen können zur Erzielung antistatischer Eigenschaften auch in thermoplastische, duroplastische oder elastomere Polymere eingearbeitet werden. Hierzu werden zweckmässig z.B. die substituierten Tetrathiobzw. Tetraselenotetracene zusammen mit einem löslichen Polymer oder einer Vorstufe davon und einem Elektronenacceptor, z.B. einem Halogen bildenden Mittel (organische halogenierte Verbindungen wie z.B. Bromoform, Trichlorbrommethan, Tetrabrommethan, Hexachlorpropan, Perchlorbutadien, 1,3-oder 1,4-Dichlor-2-buten, 1,4-Bis(trichloromethyl)-benzol, Iodacetonitril, Iodoform, Tetrachlorethylen, Perchlorcyclobutadien, N-Chlor-, -brom oder -iodsuccinimid) gegebenenfalls zusammen mit einem weiteren inerten Lösungsmittel gelöst und das überschüssige Halogen bildende Mittel und das Lösungsmittel bei höherer Temperatur verdampft. Die gebildete Zusammensetzung enthält im Polymer ein Netzwerk nadelförmiger Kristalle des CT-Komplexes, wenn das chalkogenierte Tetracen unsubstituiert ist oder kleine Substituenten (z.B. F, $CH_3$, $CF_3$) enthält. Solche Zusammensetzungen weisen eine hohe elektrische Leitfähigkeit auf. Diese kann noch verbessert werden, wenn man ein aus den Verbindungen der Formel I hergestelltes substituiertes Tetrathio- oder Tetraselenotetracen mitverwendet, das kein solches Netzwerk bildet und das in der Polymermatrix in feiner Verteilung vorliegt, da solche substituierten Tetrathio- oder Tetraselenotetracene keine oder nur eine geringe Kristallisationstendenz im Polymer besitzen. Naphthacenchinone können ferner auch in elektrochromen Displayelementen verwendet werden (JP-OS 61-43680).

Die nachfolgenden Beispiele erläutern die Erfindung.

Beispiele 1-12: 2-(2-Hydroxyethylthio-)-naphthacen-5,12-dion:

1 g (3,62 mMol) 2-Fluor-naphthacen-5,12-dion (hergestellt gemäss US-PS 4,522,754), 0,31 g (3,98 mMol) 2-Mercapto-ethanol, 1,50 g (10,86 mMol) Kaliumcarbonat und 10 ml DMSO werden während 3 Min. Bei 25°C gerührt. Das Gemisch wird auf Wasser ausgetragen. Die Kristalle werden abfiltriert und in Tetrahydrofuran/Toluol gelöst, die Lösung über Natriumsulfat getrocknet und eingedampft. Der Rückstand wird aus THF/Toluol/Pentan umkristallisiert. Ausbeute 1 g (83 %); Smp. 195-196°C.

Analog werden die in nachfolgender Tabelle 1 aufgeführten Verbindungen hergestellt:

Tabelle 1

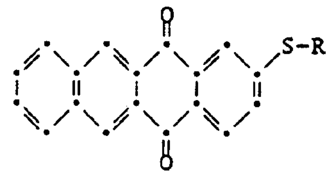

| Beispiel | R | Reaktionszeit (Minuten) | Ausbeute (%) | Schmelzpunkt (°C) |
|---|---|---|---|---|
| 2 | $-CH_3$ *) | 10 | 87 | 225-28 |
| 3 | $-CH_2-CH_3$ | 120 | 93 | 179-81 |
| 4 | $-(CH_2)_{11}-CH_3$ | 20 | 81 | 130-34 |
| 5 | $-CH_2-CH_2-COO-CH_2CHCH_2CH_2CH_2CH_3$<br>$CH_2CH_3$ | 10 | 88 | 100-103 |
| 6 | $-CH_2-COO-CH_2CHCH_2CH_2CH_2CH_3$<br>$CH_2CH_3$ | 10 | 50 | 82-84 |
| 7 | $-CH_2-COOCH_3$ | 180 | 51 | 129-30 |
| 8 | $-CH_2-CH_2-NH_2$ | 50 | 86 | 155-60 |
| 9 | $-CH_2-CH(OH)-CH_2-OH$ | 45 | 93 | 140-41 |
| 10 | $-CH_2-$ 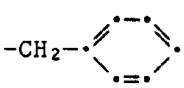 | 10 | 85 | 200-203 |
| 11 | 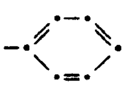 | 120 | 75 | 207-10 |
| 12 | 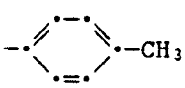 | 10 | 87 | 200-203 |

*) Hier wird anstelle des Mercaptans $NaSCH_3$ eingesetzt.

Beispiel 13: 2,8- und 2,9-Bis-(2-hydroxyethylthio-)-naphthacen-5,12-dion:

2 g (6,8 mMol) eines Gemisches von 2,8- und 2,9-Difluornaphthacen-5,12-dion werden mit 3,76 g (27,2 mMol) Kaliumcarbonat, 1 g (20,4 mMol) 2-Mercaptoethanol und 25 ml Acetonitril werden während 6,5 Stunden bei 80°C gerührt. Das Gemisch wird auf verdünnte Salzsäure ausgetragen. Der Niederschlag wird abfiltriert, dreimal mit Wasser gewaschen, dann getrocknet und aus o-Dichlorbenzol umkristallisiert; Ausbeute 2,64 g (95%) orange Kristalle vom Schmelzpunkt 180-190°C.

Das 2,8-/2,9-Difluornaphthacen-5,12-dion-Gemisch wird folgendermassen hergestellt: Eine Mischung aus 12,9 g (0,06 Mol) 6-Fluornaphthalin-2,3-dicarbonsäureanhydrid, 20,1 g $AlCl_3$ und 450 ml Fluorbenzol wird unter Rühren während einer Stunde am Rückfluss erhitzt. Nach dem Abkühlen wird das Reaktionsgemisch auf eine Mischung aus Eis und 15 ml konzentrierter HCl gegossen. Die organische Phase wird abgetrennt und im Vakuum zur Trockene eingedampft. Der Rückstand wird zwischen $CHCl_3$ und $NaHCO_3$-Lösung verteilt, die wässrige Phase mit Kohle behandelt und mit konzentrierter HCl kongorotsauer eingestellt. Der gebildete Niederschlag wird abfiltriert und getrocknet. Man erhält 13,1 g (72 %) eines 1:1-Gemisches aus 6- und 7-Fluor-3-(4-fluorbenzoyl)-2-naphthalincarbonsäure mit einem Schmelzpunkt von 251-253°C.

Dieses Gemisch wird portionenweise innerhalb 30 Minuten unter Rühren bei 145°C zu einer Schmelze aus 124,3 g $AlCl_3$ und 28 g NaCl gegeben. Die Schmelze wird 3,5 Stunden gerührt und dann auf Eis gegossen.

Der gebildete Niederschlag wird abfiltriert und getrocknet. Das erhaltene Rohprodukt (22 g) wird 20 Stunden in einer Soxhlet-Apparatur mit $CHCl_3$ extrahiert. Nach dem Abkühlen kristallisieren 4 g Produkt aus. Die Mutterlauge wird mit 1 N NaOH und $H_2O$ gewaschen und dann das Lösungsmittel verdampft. Man erhält weitere 3 g Produkt, das zur Reinigung im Hochvakuum bei 230°C sublimiert wird. Ausbeute: 6,3 g (47 %), Schmelzpunkt 275-277°C, Massenspektrum: $M^{\oplus}$ 294/100.

Nach dem gleichen Verfahren erhält man unter Verwendung von Naphthalin-2,3-dicarbonsäureanhydrid und 1,2-Difluorbenzol 2,3-Difluornaphthacen-5,12-dion.

Beispiel 14: 3-Fluor-2-methylthio-naphthacen-5,12-dion

1 g (3,4 mMol) 2,3-Difluor-naphthacen-5,12-dion, 0,94 g (6,8 mMol) Kaliumcarbonat, 0,47 g (6,8 mMol) Natrium-methanthiolat und 20 ml Tetrahydrofuran werden während 2,5 Stunden bei 25°C gerührt. Das Gemisch wird auf verdünnte Salzsäure ausgetragen und mit THF/Toluol extrahiert. Die organische Phase wird über Natriumsulfat getrocknet und eingedampft. Der Rückstand wird mit Toluol an Kieselgel chromatographiert. Ausbeute 0,55 g (50 %); Schmelzpunkt 230-35°C.

Beispiel 15: 2,3-Bis(methylthio-)-naphthacen-5,12-dion

1 g (3,4 mMol) 2,3-Difluor-naphthacen-5,12-dion, 0,94 g (6,8 mMol) Kaliumcarbonat, 0,72 g (10,2 mMol) Natrium-methanthiolat und 20 ml THF werden während 4 Stunden am Rückfluss gerührt. Das Gemisch wird auf verdünnte Salzsäure ausgetragen. Der Niederschlag wird abfiltriert, mit Wasser gewaschen und im Vakuum bei 140°C getrocknet. Durch Umkristallisation aus Dioxan wird 1 g (84 %) reines Produkt erhalten, Schmelzpunkt >260°C.

Beispiel 16:

2,3-bis(2′-Ethylhexyloxycarbonylmethylthio-)-naphthacen-5,12-dion 1 g (3,4 mMol) 2,3-Difluor-naphthacen-5,12-dion, 0,94 g (6,8 mMol) Kaliumcarbonat, 2,0 g (10,2 mMol) Thioglykolsäure-2-ethylhexyl ester und 10 ml THF werden während 20 Stunden bei 65°C gerührt. Das Gemisch wird in Wasser/THF/Toluol aufgenommen. Die organische Phase wird mit verdünnter Salzsäure, dann zweimal mit Wasser gewaschen, über Natriumsulfat getrocknet, und eingedampft. Aus Diethylether/Pentan umkristallisiert werden 2,04 g (91 %) Produkt erhalten. Schmelzpunkt 75-80°C.

Beispiele 17-20:

Analog zu Beispiel 1 wird unter Verwendung von 9-Fluor-naphthacen-5,12-dion und entsprechender Merkaptane 9-Methyl- (17), 9-Ethyl- (18), 9-Phenyl- (19) und 9-(2′-Hydroxyethyl)-naphthacen-5,12-dion (20) hergestellt.

Beispiele 21 und 22:

Gemäss Beispiel 1 werden 2,3-Di(trifluormethyl)-8,9-dibromnaphthacen-5,12-dion bzw. 8,9-Dibromnaphthacen-5,12-dion mit Thiophenol umgesetzt. Man erhält 2,3-Di(trifluormethyl)-8,9-di(phenylthio)naphthacen (Ausbeute: 65 %; Schmelzpunkt >260°C) bzw. 8,9-Di(phenylthio)naphthacen (Ausbeute: 88 %, Schmelzpunkt >260°C).

B) Anwendungsbeispiele

Beispiel 23: Photohärtung eines Acrylat-Gemisches zur Herstellung eines leliefbildes.

Es wird eine photohärtbare Zusammensetzung hergestellt durch Mischen der folgenden Komponenten:

| | | Feststoffgehalt |
|---|---|---|
| 150,30 g | Scripset 540[1)] (30 %-ige Lsg. in Aceton) | 45,1 g |
| 48,30 g | Trimethylolpropantriacrylat | 48,3 g |
| 6,60 g | Polyethylenglykoldiacrylat | 6,6 g |
| 0,08 g | Kristallviolett | |
| 205,28 g | | 100,0 g |

1) Polystyrol-Maleinsäurehalbester-Copolymer (Monsanto)

Portionen dieser Zusammensetzung werden mit 0,2 % (bezogen auf die Zusammensetzung) der in der folgenden Tabelle angegebenen Photoinitiatoren vermischt. Alle Operationen werden unter Rotlicht oder Gelblicht ausgeführt.

Die mit Initiator versetzten Proben werden mit einem Spiralrakel von 150 µm auf 200 µm Aluminiumfolie (10 x 15 cm) aufgetragen. Das Lösungsmittel wird durch Erwärmung auf 60°C während 15 Minuten im Umluftofen entfernt. Es resultiert eine Trockenschichtdicke von 35 µm. Auf die Schicht wird eine 76 µm dicke Polyesterfolie gelegt und auf diese ein standardisiertes Testnegativ mit 21 Stufen verschiedener optischer Dichte (Stouffer-Keil) gelegt. Darüber wird eine zweite Polyesterfolie gelegt und das so erhaltene Laminat mittels Vakuum auf einer Metallplatte fixiert. Die Probe wird dann mit einer 5 KW-Metallhalogenid-Lampe (Typ MO 23) im Abstand von 30 cm belichtet und zwar in einer ersten Testreihe 20 Sekunden und einer zweiten Testreihe 40 Sekunden. Nach der Belichtung werden die Folien und die Maske entfernt, die belichtete Schicht in einem Ultraschallbad 120 Sekunden mit Entwickler A 2 Minuten lang entwickelt und anschliessend bei 60°C 15 Minuten im Umluftofen getrocknet. Die Empfindlichkeit des verwendeten Initiatorsystems wird durch die Angabe der letzten klebefrei abgebildeten Keilstufe charakterisiert. Je höher die Zahl der Stufen ist, desto empfindlicher ist das System. Eine Erhöhung um zwei Stufen bedeutet dabei etwa eine Verdopplung der Härtungsgeschwindigkeit. Die Ergebnisse sind in Tabelle 2 angegeben. Entwickler A enthält 15 g Natriummetasilikat•9 $H_2O$; 0,16 g KOH; 3 g Polyethylenglykol 6000; 0,5 g Lävulinsäure und 1000 g deionisiertes Wasser.

Tabelle 2

| Photoinitiator Naphthacendion von Beispiel | Zahl der abgebildeten Stufen nach 20 s | nach 40 s Belichtung |
|---|---|---|
| 1 | 10 | 12 |
| 2 | 10 | 12 |
| 3 | 9 | 11 |
| 4 | 8 | 10 |
| 5 | 6 | 8 |
| 6 | 7 | 9 |
| 7 | 7 | 9 |
| 9 | 9 | 11 |
| 10 | 1 | 4 |
| 11 | 4 | 5 |
| 16 | 8 | 10 |
| 17 | 6 | 9 |
| 18 | 5 | 7 |
| 19 | 4 | 6 |
| 20 | 7 | 9 |

Beispiel 24:

Eine Lösung der Zusammensetzung:

| | |
|---|---|
| Ethylcellosolve | 71,00 g |
| Scripset 550*) | 14,00 g |
| Trimethylolpropantriacrylat | 15,00 g |
| Polyethylenglykol-200-diacrylat | 2,00 g |

*) Styrol-Maleinsäuremonoester-Copolymer ($M_w$ = 10'000, Säurezahl 190), Hersteller Monsanto

wird in gleiche Portionen von jeweils 10 Gramm aufgeteilt. In dieser Lösung werden jeweils 0,05 g Naphthacendion sowie 0,5 g Glycerin unter Rotlicht gelöst.

Mit einem Drahtrakel werden Beschichtungen in 12 Mikrometer Nassfilmdicke auf transparenter Polyesterfolie aufgebracht. Die nassen Filme werden in einem Umluftofen bei 80°C für 30 Minuten getrocknet.

Durch Eintauchen in eine Lösung aus

| | |
|---|---|
| Mowiol 4-88 (Polyvinylalkohol) | 30,00 g |
| Brij 35 (10 % in Wasser)**) | 15,00 g |
| deionisiertes Wasser | 250,00 g |

**) Polyoxyethylen-laurylether (Netzmittel, Hersteller Atlas Powder)

und anschliessendes Trocknen bei 80°C im Umluftofen wird eine Sauerstoffsperrschicht in 0,5 Mikrometer Dicke aufgebracht.

Der trockene Film wird mit einer 5000 W Quecksilberlampe (MO 33, Fa. Staub, Neu Isenburg) durch einen Stufenkeil mit Inkrementen von 0,15 (log O.D.) belichtet und anschliessend in einer Lösung der Zusammensetzung

| | |
|---|---|
| Natrium-metasilikat-nonahydrat | 15,00 g |
| Strontiumhydroxid-octahydrat | 0,30 g |
| Polyglykol 6000 | 3,00 g |
| Lävulinsäure | 0,50 g |
| deionisiertes Wasser | 1000,00 g |

zu einem Reliefbild entwickelt. Die Lichtintensität wird mit einem Powermeter der Fa. Oriel mit 365 nm Sensor gemessen. Tabelle 3 zeigt die für die zur Erreichung der Stufe 7 des Stufenkeils benötigte Belichtungsenergie.

Tabelle 3

| Naphthacendion | UV-Spektrum (Extinktion bei $\lambda_{max}$) | $WP_7$ ($mJ/cm^{-2}$) |
|---|---|---|
| 2-Phenylthio-naphthacen-5,12-dion | $\varepsilon$ = 11600, $\lambda_{max}$: 398 nm Schulter bei 418 nm | 26,4 |
| 2-Ethylthio-naphthacen-5,12-dion | $\varepsilon$ = 10560, $\lambda_{max}$: 398 nm Schulter bei 418 nm | 660 |

Beispiel 25: Herstellung einer Negativ-Offset-Druckplatte

Eine Beschichtungslösung aus

| | |
|---|---|
| DMI-Polymer[1]), 40 %-ig in Methoxypropanol | 2,5 g |
| Naphthacen-5,12-dion, 1 %-ig in Dimethylformamid | 5,0 ml |
| Rhodamin B (Farbstoff), 1 %-ig in Ethylcellosolve | 0,5 ml |
| Ethylglycolacetat | 2,5 ml |

[1]) Copolymer aus 86 Gew.-Teilen 1-(Dimethylmaleinimidyl)-6-methacroyl-oxy-n-hexan und 14 Gew.-Teilen Methacrylsäure, $\bar{M}_w$ = 120000 (bestimmt mit GPC, Polystyrolstandard)

wird mittels Schleuderbeschichtung (30 sec. Bei 500 U/min) auf ein Offsetplattensubstrat, d.h. elektrolytisch aufgerauhtes und anodisiertes Aluminiumblech aufgetragen und 15 min bei 80°C getrocknet. Es resultiert ein Beschichtungsgewicht von 1,1-1,3 g/m$^2$ Danach wird mit einer 2000 Watt Metallhalogenlampe während 100 sec (Abstand 75 cm von Vakuumrahmen) durch einen Stouffer-Keil (Inkremente der optischen Dichte O.D. = 0,15) belichtet. Die Entwicklung erfolgt manuell bei 20°C durch leichtes Reiben während 45 sec. mit einem Tampon, der mit folgender Entwicklerlösung getränkt ist:

75,0 g Natrium-metasilicat-pentahydrat
0,4 g Netzmittel (Supronic B 50, ABM Chemicals Ltd.)
925,0 g Deionisiertes Wasser.

In Tabelle 4 sind die verwendeten Naphtacendione und die letzte sichtbare Stufe angegeben

Tabelle 4

| Naphthacen-5,12-dion von Beispiel | Letzte sichtbare Stufe |
|---|---|
| 1 | 7 |
| 3 | 7 |
| 5 | 7 |
| 8 | 7 |
| 11 | 8-9 |

Die vernetzten Bildteile weisen eine hohe Abriebfestigkeit auf und erlauben hohe Auflagenstärken.

Beispiel 26: Sensibilisierung der 2+2-Cycloaddition

In einer 20 % (w/w) Lösung eines Copolymers mit Molekulargewicht 140'000 Dalton (gemessen durch Lichtstreuung in Dioxan bei 25°C) aus 20 Mol % Ethylacrylat und 80 Mol % N-(5-methyl-3-oxa-4-oxohexen-5-yl)-dimethylmaleinimid (hergestellt nach dem Verfahren in Angew. Makromol. Chem. 115 (1983) 163ff sowie Angew. Makromol. Chem. 128 (1984) 71ff) werden 5 % (w/w) 2-Phenylthionaphthacen-5,12-dion gelöst. Die Lösung wird als Film mit einem Drahtrakel in 14 µm Nassfilmdicke auf ein kupferbeschichtetes Glasfaser-Epoxidlaminat aufgerakelt und bei 80°C 1 Stunde getrocknet. Der trockene Film wird mit einer 5000 W Quecksilberlampe (MO 33 Fa. Staub, Neu Isenburg) durch einen Stufenkeil mit Inkrementen von 0.15 (log O.D.) belichtet und anschliessend in 1,1,1-Trichlorethan zu einem Reliefbild entwickelt. Die gemäss Beispiel 18 bestimmte

Lichtintensität $WP_7$ beträgt 165 $mJ/cm^{-2}$.

## Patentansprüche

1. Verbindungen der Formel I

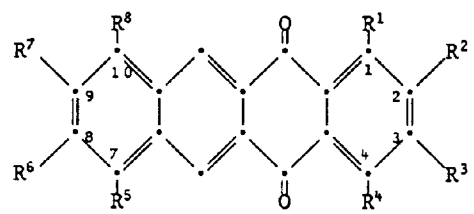

(I)

worin
$R^1$, $R^2$, $R^3$, $R^4$, $R^5$, $R^6$, $R^7$ und $R^8$ unabhängig voneinander H, -CN, $-NO_2$ Halogen, $-NR^{10}R^{11}$, $-COOR^9$, $-CONR^{10}R^{11}$, $-SiR^{13}R^{14}R^{15}$, oder unsubstituiertes oder mit -OH, -CN, $-NR^{10}R^{11}$, Halogen, $C_1$-$C_{18}$-Alkyl, -Alkoxy oder -Alkylthio, $-COOR^9$ oder $-CONR^{10}R^{11}$ substituiertes $C_1$-$C_{20}$-Alkyl$(X)_p$, $C_2$-$C_{20}$-Alkenyl$(X)_p$, $C_2$-$C_{18}$-Alkinyl$(X)_p$, $C_3$-$C_8$-Cycloalkyl$(X)_p$, $C_6$-$C_{12}$-Aryl$(X)_p$ oder $C_7$-$C_{14}$-Aralkyl$(X)_p$ darstellt oder $-Y(C_mH_{2m}O)_n R^{12}$ ist;
X für O, S, SO oder $SO_2$ und p für 0 oder 1 stehen; Y für O oder S steht;
$R^9$ H oder den um ein Hydroxylwasserstoffatom verminderten Rest eines $C_1$-$C_{20}$ -Alkohols und $R^{10}$ und $R^{11}$ unabhängig voneinander H, $C_1$-$C_{18}$-Alkyl, Phenyl, Benzyl, Cyclopentyl, Cyclohexyl, $C_2$ -$C_{12}$-Mono- oder $C_2$-$C_{12}$-Di-hydroxyalkyl, $(C_mH_{2m}O)_n$ $R^{12}$ oder $R^{10}$ und $R^{11}$ zusammen Tetra- oder Pentamethylen oder 3-Oxa-1,5-pentylen bedeuten; m 2 bis 6, n 2 bis 20 bedeuten; $R^{12}$ für H oder $C_1$-$C_{12}$-Alkyl steht; $R^{13}$, $R^{14}$ und $R^{15}$ unabhängig voneinander $C_1$-$C_{12}$-Alkyl darstellen; und wobei mindestens einer der Reste $R^2$, $R^3$, $R^5$, $R^6$, $R^7$ und $R^8$ einen Thiorest bedeuten.

2. Verbindungen gemäss Anspruch 1, worin
$R^1$ H, $-CF_3$, -OH, $C_1$-$C_{18}$-Alkyl, unsubstituiertes oder mit -OH substituiertes $C_1$-$C_{18}$-Alkoxy, unsubstituiertes oder mit Halogen, $C_1$-$C_6$-Alkyl oder -Alkoxy substituiertes $C_6$-$C_{10}$-Aryloxy, $C_7$-$C_{10}$-Aralkyloxy oder $-COOR^9$ mit $R^9$ in der Bedeutung eines um das Hydroxylwasserstoffatom verminderten $C_1$-$C_{20}$-Alkohols darstellt.

3. Verbindungen gemäss Anspruch 1, worin $R^4$, $R^5$ und $R^8$ für H stehen.

4. Verbindungen gemäss Anspruch 1, worin $R^1$, $R^4$, $R^5$ und $R^8$ für H stehen.

5. Verbindungen gemäss Anspruch 1, worin $R^2$ einen Thiorest $R^{16}$-S-bedeutet, worin $R^{16}$ für unsubstituiertes oder substituiertes $C_1$-$C_{20}$-Alkyl, Cyclopentyl, Cyclohexyl, Phenyl oder Benzyl oder $(C_mH_{2m}O)_n$ $R^{12}$ steht, wobei m eine Zahl von 2 bis 4, n eine Zahl von 2 bis 12 und $R^{12}$ H oder $C_1$-$C_{12}$-Alkyl bedeuten.

6. Verbindungen gemäss Anspruch 5, worin $R^{16}$ unsubstituiertes oder mit -OH, $-NR^{10}R^{11}$, $C_1$-$C_{18}$-Alkoxy, $-COOR^9$ oder $-CONR^{10}R^{11}$ substituiertes $C_1$-$C_{18}$-Alkyl oder $(C_mH_{2m}O)_n$ $R^{12}$ bedeutet, wobei m 2 oder 3 und n eine Zahl von 2 bis 6 sind und $R^{12}$ H oder $C_1$-$C_6$-Alkyl darstellt, wobei $R^9$, $R^{10}$ und $R^{11}$ die in Anspruch 1 angegebenen Bedeutungen haben.

7. Verbindungen gemäss Anspruch 5, worin $R^{16}$ für unsubstituiertes $C_1$-$C_{18}$-Alkyl; oder mit $C_1$-$C_{12}$-Alkoxy oder $-COOR^9$, wobei $R^9$ $C_1$-$C_{12}$-Alkyl ist, substituiertes $C_1$ bis $C_6$-Alkyl; oder mit -OH oder $-NR^{10}R^{11}$, wobei $R^{10}$ und $R^{11}$ H oder $C_1$-$C_{12}$-Alkyl sind, substituiertes $C_2$-$C_6$-Alkyl steht.

8. Verbindungen gemäss Anspruch 1, worin es sich bei dem Substituenten für $R^1$ bis $R^8$ um -OH, -F, -Cl, -Br, $-NR^{10}R^{11}$, $C_1$-$C_{12}$-Alkyl, $C_1$-$C_{12}$-Alkoxy, $C_1$-$C_{12}$-Alkylthio oder $-COOR^9$ handelt, wobei $R^9$ für H oder $C_1$-$C_{18}$-Alkyl und $R^{10}$ und $R^{11}$ unabhängig voneinander für H, $C_1$-$C_{18}$-Alkyl, $-CH_2CH_2OH$, $-CH_2CH(OH)CH_3$, $-CH_2CHOHCH_2OH$, $(C_mH_{2m}O)_n$ $R^{12}$ stehen, worin m 2 oder 3 ist, n eine Zahl von 2 bis 6 darstellt und $R^{12}$ H oder $C_1$-$C_{12}$-Alkyl bedeutet, handelt.

**9.** Verbindungen gemäss Anspruch 1, worin $R^1$ und $R^3$ bis $R^8$ unabhängig voneinander H, -CN, $-NO_2$, -F, -Cl, -Br, $-COOR^9$, $-NR^{10}R^{11}$, $-CONR^{10}R^{11}$, wobei $R^9$ H oder $C_1$-$C_{12}$-Alkyl und $R^{10}$ und $R^{11}$ unabhängig voneinander H, $C_1$-$C_{12}$-Alkyl, $-CH_2CH_2OH$, $-CH_2CH(OH)CH_3$, $-CH_2CHOHCH_2OH$ oder $-(C_mH_{2m}O)_n-R^{12}$ mit m gleich 2 oder 3, n gleich einer Zahl von 2 bis 12 und $R^{12}$ mit der Bedeutung von H oder $C_1$-$C_{12}$-Alkyl sind, oder $SiR^{13}R^{14}R^{15}$ mit $R^{13}$, $R^{14}$ und $R^{15}$ in der Bedeutung von $C_1$-$C_4$-Alkyl, oder unsubstituiertes oder substituiertes $C_1$-$C_{18}$-Alkyl$(X)_p$, $C_6H_5(X)_p$ oder $C_6H_5CH_2(X)_p$, wobei X O oder S und p 0 oder 1 bedeuten, oder $-Y-(C_mH_{2m}O)_n-R^{12}$, wobei Y O oder S, m 2 oder 3, n eine Zahl von 2 bis 12 und $R^{12}$ H oder $C_1$-$C_{12}$-Alkyl sind, darstellen.

**10.** Verbindungen gemäss Anspruch 1, worin $R^3$, oder $R^3$ und $R^6$ oder $R^7$, oder $R^3$, $R^6$ und $R^7$ die Bedeutung $R^{16}S$- haben.

**11.** Verbindungen gemäss Anspruch 1, worin $R^1$, $R^4$, $R^5$ und $R^8$ für H stehen, $R^2$, $R^3$ oder beide $-SR^{16}$ und $R^6$ und $R^7$ H, oder $R^2$, $R^6$ oder $R^7$ $-SR^{16}$ und $R^3$ und $R^7$ oder $R^3$ und $R^6$ H bedeuten.

**12.** Verbindungen gemäss Anspruch 1, worin $R^1$ und $R^3$ bis $R^8$ für H stehen und $R^2$ die Gruppe $R^{16}$ -S- bedeutet.

**13.** Verbindungen gemäss Anspruch 12, worin $R^{16}$ $C_1$-$C_{12}$-Alkyl, $C_6$-$C_{12}$-Aryl, $C_7$-$C_{18}$-Alkaryl, $-CH_2CH_2OH$, $-CH_2CHOHCH_2OH$, $-CH_2COOR^9$ oder $-CH_2CH_2COOR^9$ ist.

**14.** Verfahren zur Herstellung von Verbindungen der Formel I gemäss Anspruch 1, dadurch gekennzeichnet, dass man in einer Diels-Alder-Reaktion eine Verbindung der Formel II

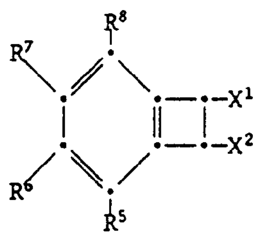

(II),

worin $R^5$, $R^6$, $R^7$ und $R^8$ die in Anspruch 1 angegebenen Bedeutungen haben und $X^1$ und $X^2$ unabhängig voneinander für -Cl, -Br - oder -J stehen, unter Abspaltung von $HX^1$ und $HX^2$ mit einer Verbindung der Formel III

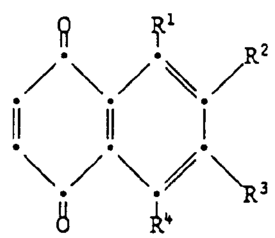

(III),

worin $R^1$, $R^2$, $R^3$ und $R^4$ die in Anspruch 1 angegebene Bedeutung haben, umsetzt.

**15.** Durch Strahlung polymerisierbare Zusammensetzung, enthaltend (a) mindestens eine nichtflüchtige, monomere, oligomere oder polymere Verbindung mit mindestens einer polymerisierbaren oder dimerisierbaren ethylenisch ungesättigten Doppelbindung und (b) mindestens eine Verbindung der Formel I nach Anspruch 1 als Photoinitiator oder Sensibilisator.

**16.** Zusammensetzungen gemäss Anspruch 15, worin die Verbindung der Formel I in einer Menge von 0,01 bis 20 Gew.-% einverleibt ist, bezogen auf die photopolymerisierbare oder photodimerisierbare Verbindung.

**17.** Beschichtetes Substrat, das auf mindestens einer Oberfläche mit einer Zusammensetzung gemäss Anspruch 15 beschichtet ist.

**18.** Verfahren zur photographischen Herstellung von Reliefabbildungen oder Beschichtungen, dadurch gekennzeichnet, dass man ein beschichtetes Substrat gemäss Anspruch 17 bildmässig oder flächenmässig belichtet und unbelichtete Anteile danach mit einem Lösungsmittel entfernt.

**19.** Verwendung von Verbindungen der Formel I gemäss Anspruch 1 als Initiatoren oder Sensibilisatoren für die Photopolymerisation oder Photodimerisation von nichtflüchtigen monomeren, oligomeren oder polymeren Verbindungen mit mindestens einer polymerisierbaren oder dimerisierbaren ethylenisch ungesättigten Doppelbindung.

**20.** Verwendung einer Zusammensetzung gemäss Anspruch 15 zur Herstellung von Lacken, Druckfarben, Druckplatten, Resistmaterialien sowie als Bildaufzeichnungsmaterial und Beschichtungsmittel.

## Claims

**1.** A compound of the formula I

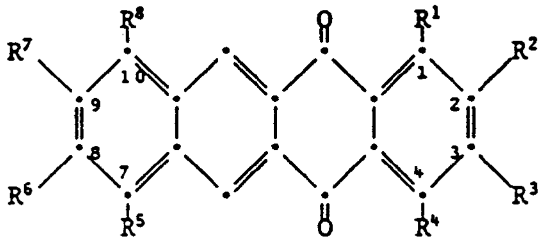

(I)

in which
$R^1$, $R^2$, $R^3$, $R^4$, $R^5$, $R^6$, $R^7$ and $R^8$, independently of one another, are H, -CN, $-NO_2$, halogen, $-NR^{10}R^{11}$, $-COOR^9$, $-CONR^{10}R^{11}$, $-SiR^{13}R^{14}R^{15}$, or unsubstituted or -OH-, -CN-, $-NR^{10}$,$R^{11}$-, halogen-, $C_1$-$C_{18}$alkyl-, -alkoxy- or -alkylthio-, $-COOR^9$- or $-CONR^{10}R^{11}$-substituted $C_1$-$C_{20}$alkyl$(X)_p$ $C_2$- $C_{20}$alkenyl $(X)_p$ , $C_2$-$C_{18}$alkynyl$(X)_p$ , $C_3$-$C_8$cycloalkyl$(X)_p$ , $C_6$-$C_{12}$aryl$(X)_p$ or $C_7$-$C_{14}$aralkyl$(X)_p$ or -Y-$(C_mH_{2m}O)_n$ $R^{12}$;
X is O, S, SO or $SO_2$ and p is 0 or 1; Y is O or S; $R^9$ is H or the radical minus a hydroxyl hydrogen atom of a $C_1$-$C_{20}$alcohol and $R^{10}$ and $R^{11}$, independently of one another, are H, $C_1$-$C_{18}$alkyl, phenyl, benzyl, cyclopentyl, cyclohexyl, $C_2$-$C_{12}$mono- or $C_2$-$C_{12}$dihydroxyalkyl, $(C_mH_{2m}O)_n$ $R^{12}$ or $R^{10}$ and $R^{11}$ together are tetra- or pentamethylene or 3-oxa-1,5-pentylene; m is 2 to 6, n is 2 to 20; $R^{12}$ is H or $C_1$-$C_{12}$alkyl; $R^{13}$, $R^{14}$ and $R^{15}$, independently of one another, are $C_1$-$C_{12}$alkyl; at least one of the radicals $R^2$, $R^3$, $R^5$, $R^6$, $R^7$ and $R^8$ being a thio radical.

**2.** A compound according to claim 1, in which $R^1$ is H, $-CF_3$, -OH, $C_1$-$C_{18}$alkyl, unsubstituted or -OH-substituted $C_1$-$C_{18}$alkoxy, unsubstituted or halogen-, $C_1$-$C_6$alkyl- or $C_1$-$C_6$alkoxy-substituted $C_6$-$C_{10}$aryloxy, $C_7$-$C_{10}$-aralkyloxy or $-COOR^9$ where $R^9$ is a $C_1$-$C_{20}$alcohol minus a hydroxyl hydrogen atom.

**3.** A compound according to claim 1, in which $R^4$, $R^5$ and $R^8$ are H.

**4.** A compound according to claim 1, in which $R^1$, $R^4$, $R^5$ and $R^8$ are H.

**5.** A compound according to claim 1, in which $R^2$ is a thio radical $R^{16}$-S-in which $R^{16}$ is unsubstituted or substituted $C_1$-$C_{20}$alkyl, cyclopentyl, cyclohexyl, phenyl or benzyl or is $(C_mH_{2m}O)_n$ $R^{12}$, m being a number from 2 to 4, n a number from 2 to 12 and $R^{12}$ H or $C_1$-$C_{12}$alkyl.

**6.** A compound according to claim 5, in which $R^{16}$ is unsubstituted or -OH-, $-NR^{10}R^{11}$-, $C_1$-$C_{18}$alkoxy-, $-COOR^9$- or $-CONR^{10}R^{11}$-subsituted $C_1$-$C_{18}$alkyl or is $(C_mH_{2m}O)_n$ $R^{12}$ in which m is 2 or 3 and n is a number from 2 to 6 and $R^{12}$ is H or $C_1$-$C_6$alkyl, $R^9$, $R^{10}$ and $R^{11}$ being as defined in claim 1.

**7.** A compound according to claim 5, in which $R^{16}$ is unsubstituted $C_1$-$C_{18}$alkyl; or $C_1$-$C_{12}$alkoxy- or $-COOR^9$-substituted $C_1$ to $C_6$alkyl in which $R^9$ is $C_1$-$C_{12}$alkyl; or -OH- or $-NR^{10}R^{11}$-substituted $C_2$-$C_6$alkyl in which $R^{10}$ and $R^{11}$ are H or $C_1$-$C_{12}$alkyl.

8. A compound according to claim 1, in which the substituents $R^1$ to $R^8$ are -OH, -F, -Cl, -Br, $-NR^{10}R^{11}$, $C_1$-$C_{12}$alkyl, $C_1$-$C_{12}$alkoxy, $C_1$-$C_{12}$alkylthio or $-COOR^9$ in which $R^9$ is H or $C_1$-$C_{18}$alkyl and $R^{10}$ and $R^{11}$, independently of one another, are H, $C_1$-$C_{18}$alkyl, $-CH_2CH_2OH$, $-CH_2CH(OH)CH_3$, $-CH_2CHOHCH_2OH$, $-(C_mH_{2m}O)_n-R^{12}$ in which m is 2 or 3, n is a number from 2 to 6 and $R^{12}$ is H or $C_1$-$C_{12}$alkyl.

9. A compound according to claim 1, in which $R^1$ and $R^3$ to $R^8$, independently of one another, are H, -CN, $-NO_2$, -F, -Cl, -Br, $-COOR^9$, $-NR^{10}R^{11}$, $-CONR^{10}R^{11}$ in which $R^9$ is H or $C_1$-$C_{12}$alkyl and $R^{10}$ and $R^{11}$, independently of one another, are H, $C_1$-$C_{12}$alkyl, $-CH_2CH_2OH$, - $CH_2CH(OH)CH_3$, $-CH_2CHOHCH_2OH$ or $-(C_mH_{2m}O)_n-R^{12}$ where m is 2 or 3, n is a number from 2 to 12 and $R^{12}$ is H or $C_1$-$C_{12}$alkyl, or $SiR^{13}R^{14}R^{15}$ where $R^{13}$ $R^{14}$ and $R^{15}$ are $C_1$-$C_4$alkyl, or unsubstituted or substituted $C_1$-$C_{18}$alkyl$(X)_p$, $C_6H_5(X)_p$ or $C_6H_5CH_2(X)_p$ in which X is O or S and p is 0 or 1, or $-Y(C_mH_{2m}O)_n-R^{12}$ in which Y is O or S, m is 2 or 3, n is a number from 2 to 12 and $R^{12}$ is H or $C_1$-$C_{12}$alkyl.

10. A compound according to claim 1, in which $R^3$, or $R^3$ and $R^6$ or $R^7$, or $R^3$, $R^6$ and $R^7$ are $R^{16}S$-.

11. A compound according to claim 1, in which $R^1$, $R^4$, $R^5$ and $R^8$ are H, $R^2$, $R^3$ or both are $-SR^{16}$ and $R^6$ and $R^7$ are H, or $R^2$, $R^6$ or $R^7$ are $-SR^{16}$ and $R^3$ and $R^7$ or $R^3$ and $R^6$ are H.

12. A compound according to claim 1, in which $R^1$ and $R^3$ to $R^8$ are H and $R^2$ is the group $R^{16}$-S-.

13. A compound according to claim 12, in which $R^{16}$ is $C_1$-$C_{12}$alkyl, $C_6$-$C_{12}$aryl, $C_7$-$C_{18}$alkaryl, $-CH_2CH_2OH$, $-CH_2CHOHCH_2OH$, $-CH_2COOR^9$ or - $CH_2CH_2COOR^9$.

14. A process for the preparation of compounds of the formula I according to claim 1 which comprises reacting in a Diels-Alder reaction a compound of the formula II

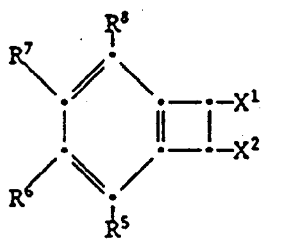

(II),

in which $R^5$, $R^6$, $R^7$ and $R^8$ are as defined in claim 1 and $X^1$ and $X^2$, independently of one another, are -Cl, -Br or -I with elimination of $HX^1$ and $HX^2$ with a compound of the formula III

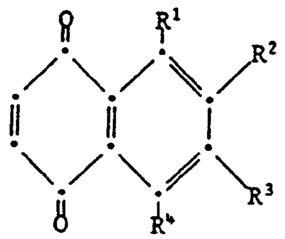

(III),

in which $R^1$, $R^2$, $R^3$ and $R^4$ are as defined in claim 1.

15. A composition polymerisable by irradiation, containing (a) at least one non volatile, monomeric, oligomeric or polymeric compound which contains at least one polymerisable or dimerisable ethylenically unsaturated double bond and (b) at least one compound of the formula I according to claim 1 as photoinitiator or sensitiser.

16. A composition according to claim 15, in which the compound of the formula I is incorporated in an amount of 0.01 to 20% by weight, relative to the photopolymerisable or photodimerisable compound.

17. A coated substrate, which is coated on at least one surface with a composition according to claim 15.

18. A process for the photographic production of relief images or coatings, which comprises subjecting a coated substrate according to claim 17 to imagewise or uniform exposure and removing unexposed portions afterwards with a solvent.

19. Use of compounds of the formula I according to claim 1 as initiators or sensitizers for the photopolymerisation or photodimerisation of non-volatile monomeric, oligomeric or polymeric compounds which contain at least one polymerisable or dimerisable ethylenically unsaturated double bond.

20. Use of a composition according to claim 15 for the preparation of paints, printing inks, printing plates, resist materials and as image recording material and coating agent.

## Revendications

1. Composés de formule I

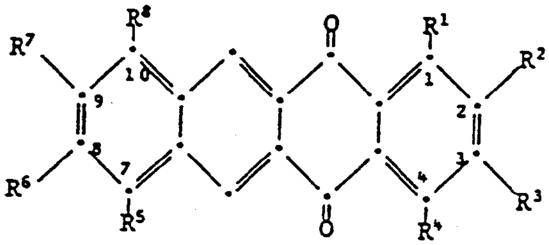

(I)

dans laquelle :

$R^2$,$R^2$, $R^3$, $R^4$, $R^5$, $R^6$, $R^7$ et $R^8$ représentent chacun , indépendamment l'un de l'autre H, -CN, $-NO_2$, un atome d'halogène, $-NR^{10}R^{11}$, $-COOR^9$, $-CONR^{10}R^{11}$, - $SiR^{12}R^{14}R^{15}$ , ou un groupe alkyle $(X)-_p$, en $C_1$ à $C_{20}$, alcényl$(X)-_p$ en $C_{2-20}$)alcynyl $(X)-_p$ en $C_2$-$C_{18}$), cycloalkyl$(X)-_p$ en $C_3$-$C_8$, aryl$(X)-_p$ en $C_{6-12}$ ou aralkyl$(X)-_p$, en $C_{7-14}$ [non substitué ou substitué par - OH, -cN, $-NR^{10}R^{11}$, un atome d'halogène, un groupe alkyle en $C_1$ à $C_{18}$, alcoxy ou alkylthio en $C_1$à $C_{18}$, $-COOR^9$ ou $-CONR^{10}R^{11}$), ou $-Y(C_mH_{2m}O)_nR^{12}$;

X représente O, S, SO ou $SO_2$, et p vaut 0 ou 1;

Y représente O, S, $R^9$ représente H ou le reste d'un alcool en $C_1$ à $C_{20}$, diminué d'un atome d'hydrogène de groupe hydroxyle, et $R^{10}$ et $R^{11}$ représentent chacun, indépendamment l'un de l'autre, H, un groupe alkyle en $C_1$ à $C_{18}$, phényle,benzyle, cyclopentyle, cyclohexyle, monohydroxyalkyle (en $C_2$ à $C_{12}$) ou dihydroxyalkyle (en $C_2$ à $C_{12}$ par groupe alkyle) , $(-C_mH_{2m}O)_nR^{12}$ ou bien $R^{10}$ et $R^{11}$ forment ensemble un groupe tétraméthylène ou pentaméthylène ou 3-oxa-pentylène; m vaut 2 à 6, n vaut 2 à 20; $R^{12}$ représente H ou un groupe alkyle en $C_1$ à $C_{12}$; $R^{13}$, $R^{14}$ et $R^{15}$, représentent chacun, indépendamment l'un de l'autre, un groupe alkyle en $C_1$ à $C_{12}$; et au moins l'un des restes $R^2$, $R^3$, $R^5$, $R^6$, $R^7$ et $R^8$ est un reste thio .

2. Composés selon la revendication 1 , dans lesquels

$R^1$ représente H,$-CF_3$, OH, un groupe alkyle en $C_1$ à $C_{18}$, un groupe alcoxy en $C_1$ à $C_{18}$ (non substitué ou substitué par -OH), un groupe aryloxy en $C_6$ à $C_{10}$ (non substitué ou substitué par de l'halogène, par un groupe alkyle ou alcoxy en $C_1$ à $C_6$), un groupe aralkyloxy en $C_7$ à $C_{10}$ ou $-COOR^9$, $R^9$ représentant un alcool en $C_1$à $C_{20}$ dont un atome d'hydrogène de groupe hydroxyle a été enlevé.

3. Composés selon la revendication 1, dans lesquels les symboles $R^4$, $R^5$ et $R^6$ représentent chacun H.

4. Composés selon la revendication 1, dans lesquels les symboles $R^1$, $R^4$, $R^5$, $R^8$ représentent chacun H,

5. Composés selon la revendication 1, dans lesquels $R^2$ représente un reste thio $R^{16}$-S-, dans lequel $R^{16}$ représente un groupe (substitué ou non substitué) alkyle en $C_1$ à $C_{20}$, cyclopentyle, cyclohexyle, phényle ou benzyle ou $(C_mH_{2m}O)_n$ $R^{12}$, où m est un nombre valant 2 à 4, n est un nombre valant 2 à 12, et $R^{12}$ représente H ou un groupe alkyle en $C_1$ à $C_{12}$.

6. Composés selon la revendication 5, dans lesquels $R^{16}$ représente un groupe alkyle en $C_1$ à $C_{18}$ (non subs-

titué ou substitué) par -OH, $NR^{10}R^{11}$, un groupe alcoxy en $C_1$ à $C_{18}$ $-COOR^9$ ou $-CONR^{10}R^{11}$), ou $(C_mH_{2m}O)_n-R^{12}$, où m vaut 2 ou 3, et n est un nombre valant 2 à 6; et $R^{12}$ représente H ou un groupe alkyle en $C_1$ à $C_6$, les symboles $R^9$, $R^{10}$ et $R^{11}$ ayant les sens indiqués à la revendication 1.

7. Composés selon la revendication 5, dans lesquels $R^{16}$ représente un groupe alkyle en $C_1$ à $C_{18}$, qui n'est pas substitué; ou un groupe alkyle en $C_1$ à $C_6$, (substitué par un groupe alcoxy en $C_1$ à $C_{12}$) ou par $-COOR^9$, le symbole $R^9$ représentant un groupe alkyle en $C_1$ à $C_{12}$) ou un groupe alkyle en $C_2$ à $C_6$ (substitué par un -OH ou par - $NR^{10}R^{11}$, les symboles $R^{10}$ et $R^{11}$ représentant chacun H ou un groupe alkyle en $C_1$ à $C_{12}$).

8. Composés selon la revendication 1, dans lesquels il s'agit, pour les susbtituants représentés par 1 à $R^8$, d'un groupe -OH,-F, -Cl, -Br, $-NR^{10}R^{11}$, alkyle en $C_1$ à $C_{12}$, alcoxy en $C_1$ à $C_{12}$, alkylthio en $C_1$ à $C_{12}$, ou $-COOR^9$ le symbole $R^9$ représentant H ou un groupe alkyle en $C_1$ à $C_{18}$, et $R^{10}$ et $R^{11}$ représentant chacun, indépendamment l'un de l'autre, H, un groupe alkyle en $C_1$ à $C_{18}$, $-CH_2CH_2OH$, - $CH_2CH(OH)CH_3$, - $CH_2CHOHCH_2OH$, $(C_mH_{2m}O)_n$ $R^{12}$, l'indice m valant 2 ou 3, et n étant un nombre valant 2 à 6, et $R^{12}$ représentant H ou un groupe alkyle en $C_1$ à $C_{12}$.

9. Composés selon la revendication 1, dans lesquels $R^1$ et $R^3$ à $R^8$ représentent chacun, indépendamment l'un de l'autre, H-CN, -NO -F, -Cl, -Br, $-COOR^9$, $-NR^{10}R^{11}$ $-CONR^{10}R^{11}$, le symbole $R^9$ représentant H ou un groupe alkyle en $C_1$ à $C_{12}$ et les symboles $R^{10}$ et $R^{11}$ représentant chacun, indépendamment l'un de l'autre, H, un groupe alkyle en $C_1$ à $C_{12}$, $-CH_2CH_2OH$, $-CH_2CH(OH)CH_3$, $-CH_2CHOHCH_2OH$ ou $(C_mH_{2m}O)$ $_nR^{12}$, m valant 2 ou 3, n étant un nombre valant 2 à 12, et $R^{12}$ représentant H ou un groupe alkyle en $C_1$ à $C_{12}$ ou bien $SiR^{13}R^{14}R^{15}$ les symboles $R^{13}$, $R^{14}$ et $R^{15}$ représentant chacun un groupe alkyle en $C_1$ à $C_4$, ou un groupe alkyle $(X)-_p$ en $C_1$ à $C_{18}$, $C_6H_5$ $(X)-_p$ ou C $_6H_5CH_2(X)_p$ (substitués ou non substitués), le symbole X représentant O ou S et p valant 0 ou 1, ou bien -Y $(C_mH_{2m}O)$ $_nR^{12}$, le symbole Y représentant O ou S, m valant 2 ou 3, n étant un nombre valant 2 à 12, et $R^{12}$ représentant H ou un groupe alkyle en $C_1$ à $C_{12}$.

10. Composés selon la revendication 1,dans lesquels $R^3$ ou bien $R^3$ et $R^6$ ou $R^7$ ou bien $R^3$, $R^6$ et $R^7$ représentent $R^{16}S$-.

11. Composés selon la revendication 1, dans lesquels, $R^1$, $R^4$, $R^5$ et $R^7$ représentent chacun H, $R^2$, $R^3$ ou les deux représentent chacun $-SR^{16}$, et $R^6$ et $R^7$ représentent chacun H ou bien $R^2$, $R^6$ et $R^7$ représentent chacun $-SR^{14}$ et $R^3$ et $R^7$ ou bien $R^3$ et $R^6$ représentent chacun H.

12. Composés selon la revendication 1, dans lesquels $R^1$ et $R^3$ à $R^8$ représentant chacun H, et $R^7$ représente le groupe $R^{16}$-S-.

13. Composés selon la revendication 12, dans lesquels $R^{16}$ représente un groupe alkyle en $C_1$ à $C_{12}$ aryle en $C_6$ à $C_{12}$, alcaryle en $C_7$ à $C_{18}$, $-CH_2CH_2OH$, $-CH_2CHOHCH_2OH$, $-CH_2COOR^9$ ou $CH_2CH_2COOR^9$.

14. Procédés pour préparer des composés de formule I selon la revendication 1, caractérisés en ce qu'on fait réagir, dans une réaction de Diels-Alder, un composé de formule II

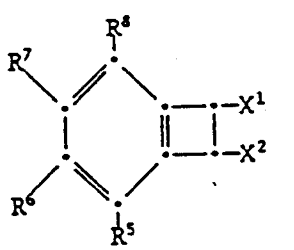

(II),

(dans laquelle $R^5$, $R^6$, $R^7$ et $R^8$ ont les sens indiqués à la revendication 1; et $X^1$ et $X^2$ représentent chacun , indépendamment l'un de l'autre, -Cl, -Br, ou -I), avec séparation de $HX^1$ et de $HX^2$, avec un composé de formule III

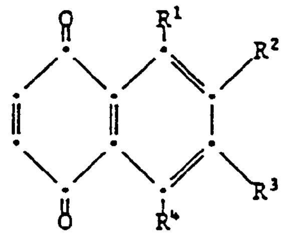

(III),

dans laquelle $R^1$, $R^2$, $R^3$ et $R^4$ ont le sens indiqué à la revendication 1.

**15.** Composition polymérisable sous l'effet d'un rayonnement, cette composition contenant (a) au moins un composé monomère, oligomère ou polymère non volatil comportant au moins une double liaison d'insaturation éthylénique polymérisable ou dimérisable et, (b) au moins un composé de formule 1 selon la revendication 1 comme photoamorceur ou sensibilisateur.

**16.** Compositions selon la revendication 15, dans lesquelles le composé de formule I est incorpore en une quantité de 0, 01 à 2O % en poids, par rapport au composé photopolymérisable ou photodimérisable .

**17.** Substrat revêtu, qui est revêtu sur une face au moins par une composition selon la revendication 15.

**18.** Procédé de préparation photographique d'image en relief ou de revêtements, procédé caractérisé en ce qu'on expose, de façon à former une image ou sur la surface complète, un susbtrat revêtu selon la revendication 17 et l'on enlève, ensuite, à l'aide d'un solvant, les parties non exposées ou non éclairées.

**19.** Utilisation de composés de la formule I selon la revendication 1 comme amorceurs ou sensibilisateurs pour la photopolymérisation ou la photodimérisation de composés monomères, oligomères ou polymères non volatils, comportant au moins une double liaison d'insaturation éthylénique polymérisable ou dimérisable.

**20.** Utilisation d'une composition selon la revendication 15 pour préparer des vernis, des encres d'impression, des plaques d'impression, des matières pour caches ou réserves ainsi que comme matériau pour réception d'images et produits pour revêtement.